# EUROPEAN PATENT APPLICATION

(11) **EP 4 484 415 A1**
(43) Date of publication of application: **01.01.2025**
(21) Application number: 23759208.4
(22) Date of filing: 22.02.2023
(51) Int. Cl.: C07D 295/135, A61K 31/496, A61K 9/16, A61P 25/18, A61P 25/28

(54) **MEDICINAL SALT OF CARIPRAZINE AND CRYSTAL FORM THEREOF, PHARMACEUTICAL COMPOSITION THEREOF, AND PREPARATION METHOD THEREFOR AND USE THEREOF**

(30) Priority: 22.02.2022 CN 202210162007
(71) Applicant: Shanghai Yonsun Biotechnology Co., Ltd., Shanghai 201210 (CN)
(72) Inventor: CHEN, Zhixiang, Shanghai 201210 (CN); ZHANG, Xian, Shanghai 201210 (CN); ZHU, Tao, Shanghai 201210 (CN); FU, Jun, Shanghai 201210 (CN); GUO, Zhen, Shanghai 201210 (CN); WANG, Tingting, Shanghai 201210 (CN); YING, Shuhuan, Shanghai 201210 (CN)
(74) Representative: Gille Hrabal Partnerschaftsgesellschaft mbB Patentanwälte
(86) International application number: PCT/CN2023/077671
(87) International publication number: WO 2023/160583

(57) **Abstract**

Disclosed are 1-hydroxy-2-naphthoate or bis-hydroxynaphthenate of cariprazine, a new solid form thereof, a pharmaceutical composition thereof, and a preparation method therefor and the use thereof. The cariprazine salt and the solid form thereof, especially 1-hydroxy-2-naphthoate of cariprazine in an insoluble salt of cariprazine has the advantages of a long-acting sustained-release preparation, can be developed into a suspension injection, can also solve the problems of the risk of the dissociation or the lack of sustained release effect of existing salt types such as cariprazine hydrochloride, can achieve the effect of long-acting administration, and greatly improves the compliance of a patient and the bioavailability and medication safety of a drug.

## Description

The present application claims priority to the prior application with the application No. 202210162007.8 entitled "MEDICINAL SALT OF CARIPRAZINE AND CRYSTAL FORM THEREOF, PHARMACEUTICAL COMPOSITION THEREOF, AND PREPARATION METHOD THEREFOR AND USE THEREOF" and filed with the China National Intellectual Property Administration on February 22, 2022, which is incorporated herein by reference in its entirety.

### TECHNICAL FIELD

The present disclosure belongs to the field of chemical pharmaceuticals, and particularly relates to a pharmaceutically acceptable salt of cariprazine, a crystal form thereof, a pharmaceutical composition thereof, a preparation method therefor and use thereof.

### BACKGROUND

Cariprazine with a chemical structure shown below is a novel atypical antipsychotic that has an antagonistic effect on dopamine D3 receptor, dopamine D2 receptor and 5-hydroxytryptamine 2B receptor. It can be used for treating schizophrenia and bipolar I disorder. Cariprazine hydrochloride capsules are currently available on the market. This product is an oral preparation that should be taken daily to maintain its plasma concentration. However, the patient compliance is poor due to required frequent administration.

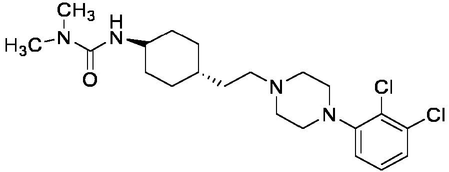

The patent CN101679315A discloses various salts of cariprazine, including monohydrochloride, dihydrochloride, monohydrobromide, maleate and mesylate.

The patent CN105218484A discloses cariprazine tartrate, and provides the solubilities of cariprazine tartrate, cariprazine hydrochloride, cariprazine maleate, cariprazine benzenesulfonate and cariprazine phosphate, all of which are greater than 3 mg/mL.

The patent WO2020056929A discloses a new crystal form of cariprazine hydrochloride and mentions that cariprazine hydrochloride dissociates rapidly into a free base in a pH 6.5 buffer.

The patent CN108261394A discloses an injectable preparation of cariprazine hydrochloride including aqueous suspension form and lyophilizate form, which can achieve a sustained release over at least 1 week or longer. However, the inventors found during research that cariprazine hydrochloride is not very stable in an aqueous solution, that is, it will dissociate under slightly acidic to alkaline conditions, and therefore it may dissociate in aqueous suspension, changing the properties and quality of the product and the dissolution and absorption of the drug, and influencing the therapeutic effect of the drug and its medication safety in patients, which is not suitable for use in long-acting sustained-release preparations. Moreover, the administration concentration of cariprazine in the injectable preparation of cariprazine hydrochloride and the plasma concentration in the pK experiments on animals disclosed in the patent CN108261394A are too high. Given that cariprazine clearance considerably varies in species as shown by original research data (about 2-4 hours in rats, 3-9 days in humans), an excessive plasma concentration may cause stronger toxic and side effects and excessive drug accumulation in the human body. No injectable preparations of very slightly soluble salts of cariprazine with improved properties have now been reported.

In view of the defect in the prior art, the search for a pharmaceutically acceptable salt of cariprazine and crystal forms thereof which have low solubility, high stability and good clinical effect and are suitable for long-acting administration and/or commercialization is an urgent technical problem in need of solution in the art.

### SUMMARY

In order to solve the problems described above in the prior art, the present disclosure provides a pharmaceutically acceptable salt of cariprazine, especially cariprazine 1-hydroxy-2-naphthoate or cariprazine embonate, and/or a new solid form thereof, as well as a pharmaceutical composition thereof, a preparation method therefor and use thereof.

According to an embodiment of the present disclosure, the cariprazine 1-hydroxy-2-naphthoate is selected from cariprazine mono-1-hydroxy-2-naphthoate represented by formula (I) shown below, or cariprazine bis-1-hydroxy-2-naphthoic acid represented by formula (II) shown below:

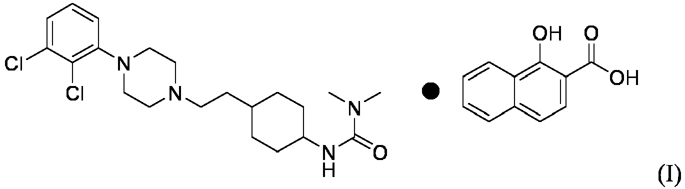

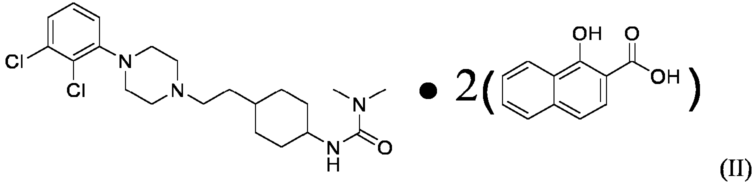

According to an embodiment of the present disclosure, the cariprazine 1-hydroxy-2-naphthoate is crystal form A of cariprazine mono-1-hydroxy-2-naphthoate, preferably crystal form A of cariprazine mono-1-hydroxy-2-naphthoate hydrate, and more preferably crystal form A of cariprazine mono-1-hydroxy-2-naphthoate dihydrate; wherein the crystal form A of cariprazine mono-1-hydroxy-2-naphthoate, preferably the crystal form A of cariprazine mono-1-hydroxy-2-naphthoate hydrate, and more preferably the crystal form A of cariprazine mono-1-hydroxy-2-naphthoate dihydrate, has an X-ray powder diffraction pattern comprising characteristic peaks at 20 values of 15.6°±0.2°, 19.7°±0.2°, 20.7°±0.2°, etc.

Further, the crystal form A of cariprazine mono-1-hydroxy-2-naphthoate, preferably the crystal form A of cariprazine mono-1-hydroxy-2-naphthoate hydrate, and more preferably the crystal form A of cariprazine mono-1-hydroxy-2-naphthoate dihydrate, has an X-ray powder diffraction pattern comprising characteristic peaks at 20 values of 12.7°±0.2°, 15.6°±0.2°, 17.3°±0.2°, 19.7°±0.2°, 20.2°±0.2°, 20.7°±0.2°, etc.

Further, the crystal form A of cariprazine mono-1-hydroxy-2-naphthoate, preferably the crystal form A of cariprazine mono-1-hydroxy-2-naphthoate hydrate, and more preferably the crystal form A of cariprazine mono-1-hydroxy-2-naphthoate dihydrate, has an X-ray powder diffraction pattern comprising characteristic peaks at 20 values of 12.7°±0.2°, 15.6°±0.2°, 17.3°±0.2°, 18.8°±0.2°, 19.7°±0.2°, 20.2°±0.2°, 20.7°±0.2°, 24.3°±0.2°, etc.

Furthermore, the crystal form A of cariprazine mono-1-hydroxy-2-naphthoate, preferably the crystal form A of cariprazine mono-1-hydroxy-2-naphthoate hydrate, and more preferably the crystal form A of cariprazine mono-1-hydroxy-2-naphthoate dihydrate, has an X-ray powder diffraction pattern comprising absorption peaks at 20 values of 4.4°±0.2°, 8.6°±0.2°, 9.7°±0.2°, 11.4°±0.2°, 12.7°±0.2°, 15.6°±0.2°, 16.5°±0.2°, 16.9°±0.2°, 17.3°±0.2°, 18.8°±0.2°, 19.7°±0.2°, 20.2°±0.2°, 20.7°±0.2°, 23.3°±0.2°, 24.3°±0.2°, 24.9°±0.2°, 29.5°±0.2°, etc.

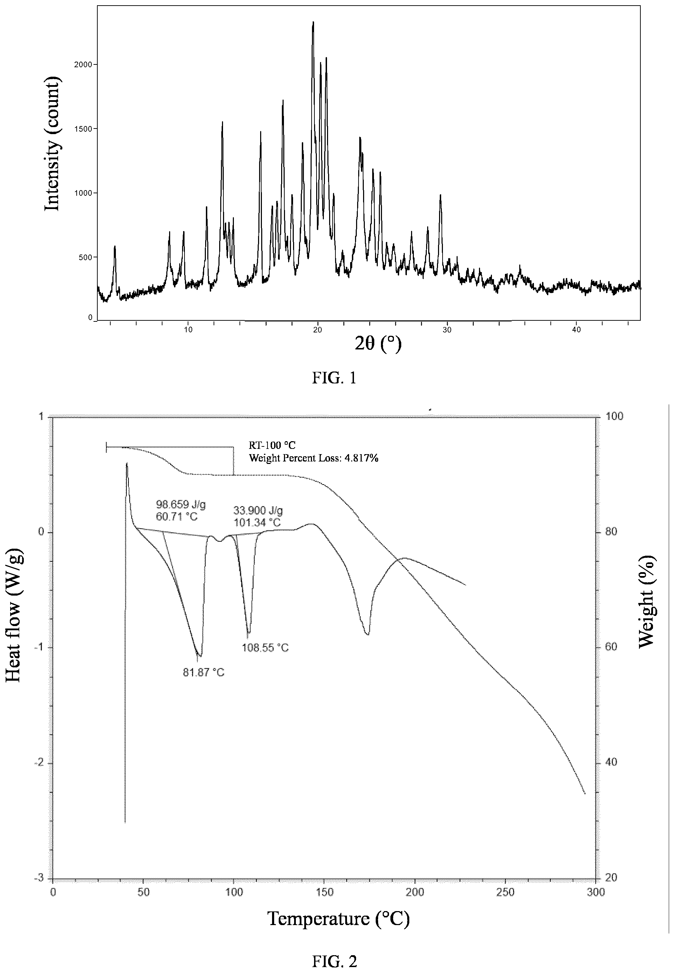

Furthermore, the crystal form A of cariprazine mono-1-hydroxy-2-naphthoate, preferably the crystal form A of cariprazine mono-1-hydroxy-2-naphthoate hydrate, and more preferably the crystal form A of cariprazine mono-1-hydroxy-2-naphthoate dihydrate, has an X-ray powder diffraction pattern substantially as shown in FIG. 1.

Preferably, the crystal form A of cariprazine mono-1-hydroxy-2-naphthoate, preferably the crystal form A of cariprazine mono-1-hydroxy-2-naphthoate hydrate, and more preferably the crystal form A of cariprazine mono-1-hydroxy-2-naphthoate dihydrate, has a differential scanning calorimetry pattern substantially as shown in FIG. 2.

Preferably, the crystal form A of cariprazine mono-1-hydroxy-2-naphthoate, preferably the crystal form A of cariprazine mono-1-hydroxy-2-naphthoate hydrate, and more preferably the crystal form A of cariprazine mono-1-hydroxy-2-naphthoate dihydrate, has a melting point of about 101.3 °C.

Preferably, the crystal form A of cariprazine mono-1-hydroxy-2-naphthoate, preferably the crystal form A of cariprazine mono-1-hydroxy-2-naphthoate hydrate, and more preferably the crystal form A of cariprazine mono-1-hydroxy-2-naphthoate dihydrate, has a thermogravimetric analysis pattern substantially as shown in FIG. 2, indicating a weight loss of about 4.82% before 100 °C.

Preferably, the crystal form A of cariprazine mono-1-hydroxy-2-naphthoate is crystal form A of cariprazine mono-1-hydroxy-2-naphthoate hydrate, more preferably of cariprazine mono-1-hydroxy-2-naphthoate dihydrate.

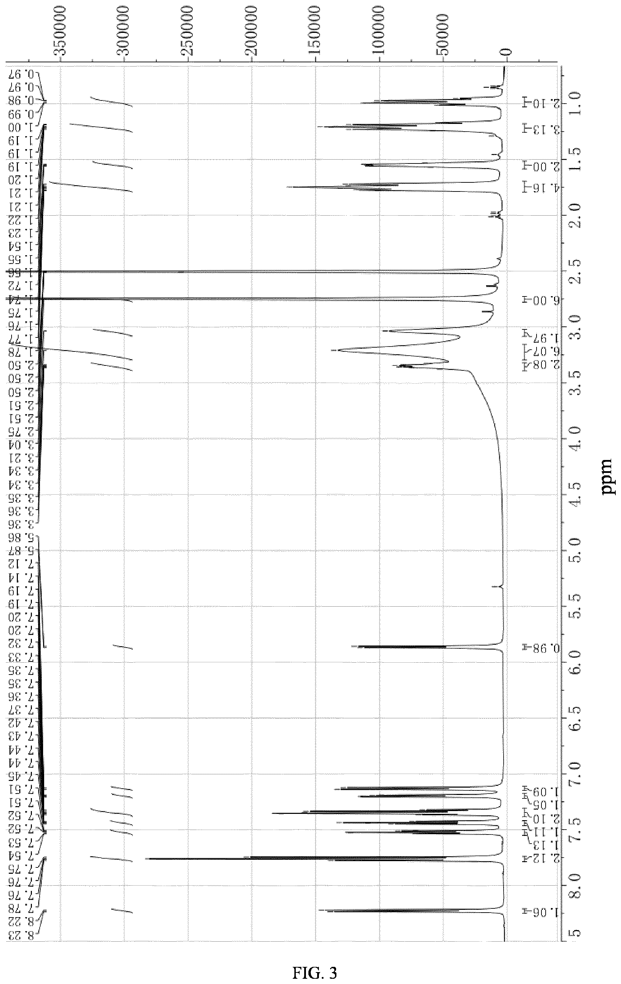

Furthermore, in the crystal form A of cariprazine mono-1-hydroxy-2-naphthoate, preferably the crystal form A of cariprazine mono-1-hydroxy-2-naphthoate hydrate, and more preferably the crystal form A of cariprazine mono-1-hydroxy-2-naphthoate dihydrate, cariprazine and 1-hydroxy-2-naphthoic acid form the salt in a molar ratio of 1:1, and a ¹H-NMR spectrum thereof is substantially as shown in FIG. 3.

The present disclosure further provides a single crystal of crystal form A of cariprazine mono-1-hydroxy-2-naphthoate dihydrate, and the single crystal has an X-ray diffraction pattern comprising characteristic peaks at 20 values of 17.5°±0.2°, 19.7°±0.2°, 20.8°±0.2°, etc.

Further, the single crystal has an X-ray diffraction pattern comprising characteristic peaks at 20 values of 17.5°±0.2°, 19.0°±0.2°, 19.7°±0.2°, 20.3°±0.2°, 20.8°±0.2°, 23.4°±0.2°, etc.

Further, the single crystal has an X-ray diffraction pattern comprising characteristic peaks at 20 values of 12.7°±0.2°, 15.7°±0.2°, 17.5°±0.2°, 19.0°±0.2°, 19.7°±0.2°, 20.3°±0.2°, 20.8°±0.2°, 23.4°±0.2°, etc.

Furthermore, the single crystal has an X-ray diffraction pattern comprising absorption peaks at 20 values of 11.5°±0.2°, 12.7°±0.2°, 15.7°±0.2°,, 16.6°±0.2°, 16.9°±0.2°, 17.5°±0.2°, 18.1°±0.2°, 19.0°±0.2°, 19.7°±0.2°, 20.0°±0.2°, 20.3°±0.2°, 20.8°±0.2°, 21.3°±0.2°, 23.4°±0.2°, 23.6°±0.2°, 24.4°±0.2°, 24.9°±0.2°, 29.6°±0.2°, etc.

According to an embodiment of the present disclosure, further provided is a preparation method for the crystal form A of cariprazine mono-1-hydroxy-2-naphthoate, preferably the crystal form A of cariprazine mono-1-hydroxy-2-naphthoate hydrate, and more preferably the crystal form A of cariprazine mono-1-hydroxy-2-naphthoate dihydrate, comprising the following steps:
(a1) dissolving cariprazine in an aqueous phosphoric acid solution and filtering to obtain solution A; mixing an aqueous 1-hydroxy-2-naphthoic acid solution with an aqueous sodium hydroxide solution and filtering to obtain solution B; and
(a2) adding the solution B to the solution A according to a solute molar ratio of 1:1 and stirring at room temperature to obtain the crystal form A of cariprazine mono-1-hydroxy-2-naphthoate, preferably the crystal form A of cariprazine mono-1-hydroxy-2-naphthoate hydrate, and more preferably the crystal form A of cariprazine mono-1-hydroxy-2-naphthoate dihydrate.

Alternatively, the preparation method for the crystal form A of cariprazine mono-1-hydroxy-2-naphthoate, preferably the crystal form A of cariprazine mono-1-hydroxy-2-naphthoate hydrate, and more preferably the crystal form A of cariprazine mono-1-hydroxy-2-naphthoate dihydrate, comprises the following steps:
(a3) mixing cariprazine and 1-hydroxy-2-naphthoic acid with a solvent according to a molar ratio of 1:1; and
(a4) stirring at 25-70 °C to obtain the crystal form A of cariprazine mono-1-hydroxy-2-naphthoate, preferably the crystal form A of cariprazine mono-1-hydroxy-2-naphthoate hydrate, and more preferably the crystal form A of cariprazine mono-1-hydroxy-2-naphthoate dihydrate. According to an embodiment of the present disclosure, the solvent is a mixed system of methanol, ethanol or acetone and water. Preferably, methanol, ethanol or acetone and water may be in a volume ratio of 2:1 to 1:2, such as 1:1.

Preferably, cariprazine and the total volume of the solvent system are in a mass-to-volume ratio of 1 g:(5-30) mL, such as 1 g:(5-20) mL, e.g., 1 g:10 mL.

According to an embodiment of the present disclosure, the cariprazine 1-hydroxy-2-naphthoate is crystal form B of cariprazine mono-1-hydroxy-2-naphthoate, wherein the crystal form B of cariprazine mono-1-hydroxy-2-naphthoate has an X-ray powder diffraction pattern comprising characteristic peaks at 20 values of 14.6°±0.2°, 18.8°±0.2°, 19.5°±0.2°, etc.

Further, the crystal form B of cariprazine mono-1-hydroxy-2-naphthoate has an X-ray powder diffraction pattern comprising characteristic peaks at 20 values of 5.5°±0.2°, 11.5°±0.2°, 14.6°±0.2°, 18.8°±0.2°, 19.5°±0.2°, 23.7°±0.2°, etc.

Further, the crystal form B of cariprazine mono-1-hydroxy-2-naphthoate has an X-ray powder diffraction pattern comprising characteristic peaks at 20 values of 5.5°±0.2°, 11.5°±0.2°, 13.0°±0.2°, 14.6°±0.2°, 16.9°±0.2°, 18.8°±0.2°, 19.5°±0.2°, 23.7°±0.2°, etc.

Furthermore, according to an embodiment of the present disclosure, the crystal form B of cariprazine mono-1-hydroxy-2-naphthoate has an X-ray powder diffraction pattern comprising characteristic peaks at 20 values of 5.5°±0.2°, 9.2°±0.2°, 11.1°±0.2°, 11.5°±0.2°, 13.0°±0.2°, 13.9°±0.2°, 14.6°±0.2°, 16.6°±0.2°, 16.9°±0.2°, 18.5°±0.2°, 18.8°±0.2°, 19.5°±0.2°, 19.9°±0.2°, 20.3°±0.2°, 21.2°±0.2°, 23.7°±0.2°, 24.3°±0.2°, etc.

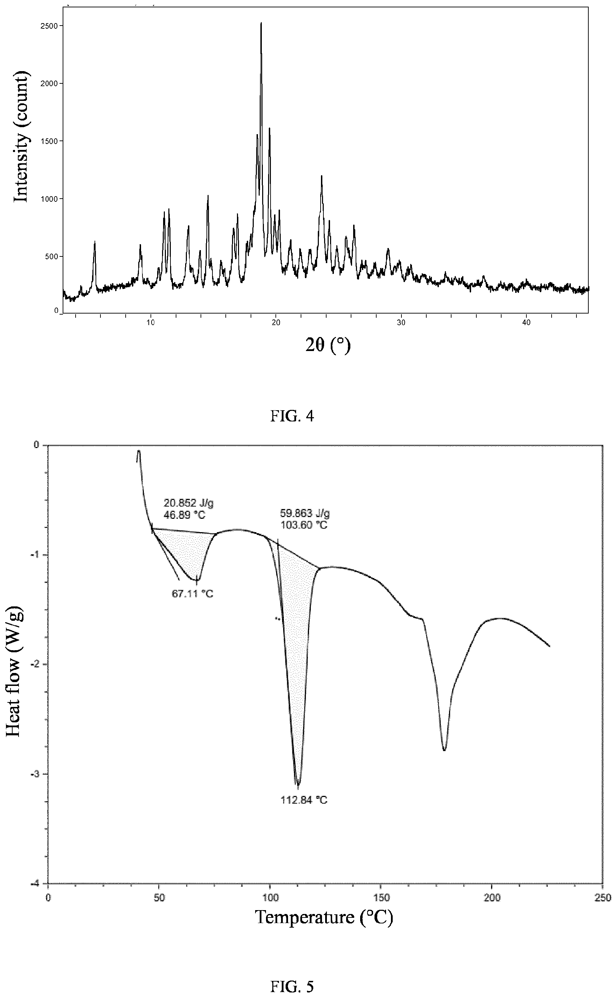

Furthermore, the crystal form B of cariprazine mono-1-hydroxy-2-naphthoate has an X-ray powder diffraction pattern substantially as shown in FIG. 4.

Preferably, the crystal form B of cariprazine mono-1-hydroxy-2-naphthoate has a differential scanning calorimetry pattern substantially as shown in FIG. 5.

Preferably, the crystal form B of cariprazine mono-1-hydroxy-2-naphthoate has a melting point of about 106.8 °C.

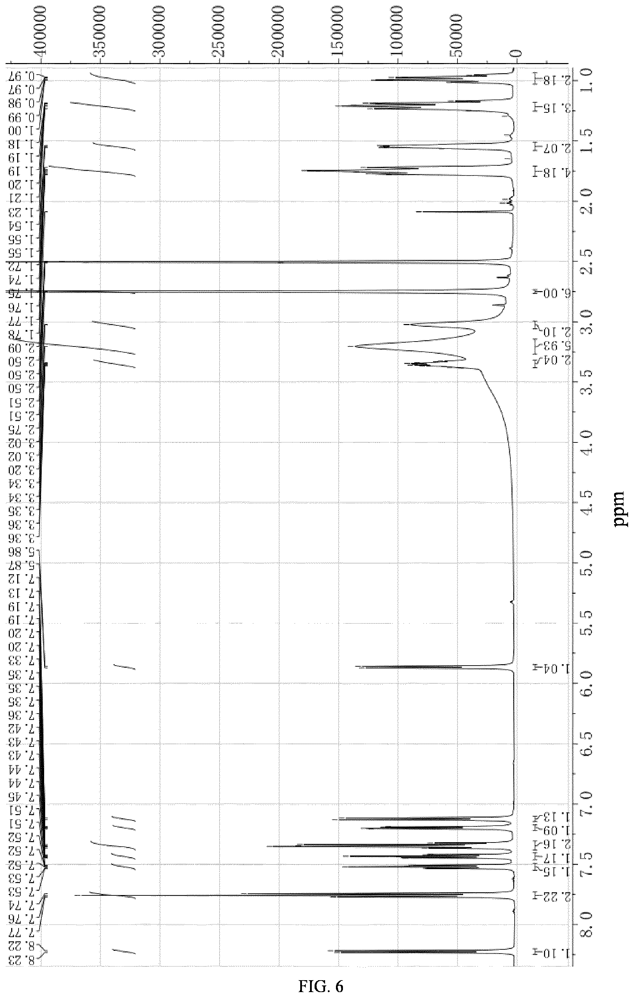

Furthermore, in the the crystal form B of cariprazine mono-1-hydroxy-2-naphthoate, cariprazine and 1-hydroxy-2-naphthoic acid form the salt in a molar ratio of 1:1, and a ¹H-NMR spectrum thereof is substantially as shown in FIG. 6.

According to an embodiment of the present disclosure, a preparation method for the crystal form B of cariprazine mono-1-hydroxy-2-naphthoate comprises the following steps:
(b1) mixing cariprazine and 1-hydroxy-2-naphthoic acid with a solvent according to a molar ratio of 1:1; and
(b2) stirring at 25-70 °C to obtain the crystal form B of cariprazine mono-1-hydroxy-2-naphthoate.

According to an embodiment of the present disclosure, the solvent is a mixed system of methanol, ethanol or acetone and water. Preferably, methanol, ethanol or acetone and water may be in a volume ratio of 2:1 to 1:2, such as 1.5:1.

Preferably, cariprazine and the total volume of the solvent system are in a mass-to-volume ratio of 1 g:(0.5-10) mL, such as 1 g:(0.5-1.5) mL, e.g., 1 g: 1 mL.

According to an embodiment of the present disclosure, the cariprazine 1-hydroxy-2-naphthoate is crystal form C of cariprazine mono-1-hydroxy-2-naphthoate, wherein the crystal form C of cariprazine mono-1-hydroxy-2-naphthoate has an X-ray powder diffraction pattern comprising characteristic peaks at 20 values of 17.8°±0.2°, 19.9°±0.2°, 21.1°±0.2°, etc.

Further, the crystal form C of cariprazine mono-1-hydroxy-2-naphthoate has an X-ray powder diffraction pattern comprising characteristic peaks at 20 values of 4.4°±0.2°, 15.6°±0.2°, 17.8°±0.2°, 19.9°±0.2°, 21.1°±0.2°, 23.7°±0.2°, etc.

Further, the crystal form C of cariprazine mono-1-hydroxy-2-naphthoate has an X-ray powder diffraction pattern comprising characteristic peaks at 20 values of 4.4°±0.2°, 12.8°±0.2°, 15.6°±0.2°, 17.8°±0.2°, 19.9°±0.2°, 20.2°±0.2°, 21.1°±0.2°, 23.7°±0.2°, etc.

Furthermore, according to an embodiment of the present disclosure, the crystal form C of cariprazine mono-1-hydroxy-2-naphthoate has an X-ray powder diffraction pattern comprising characteristic peaks at 20 values of 4.4°±0.2°, 8.6°±0.2°, 9.7°±0.2°, 11.5°±0.2°, 12.8°±0.2°, 13.2°±0.2°, 15.6°±0.2°, 16.6°±0.2°, 17.8°±0.2°, 19.3°±0.2°, 19.9°±0.2°, 20.2°±0.2°, 21.1°±0.2°, 22.4°±0.2°, 23.7°±0.2°, 24.2°±0.2°, 24.9°±0.2°, etc.

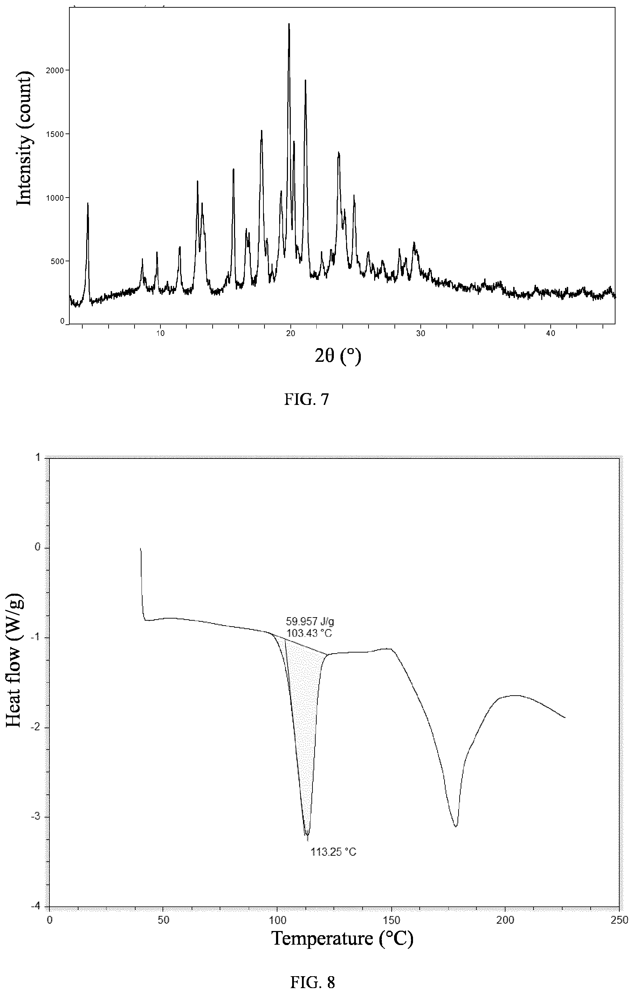

Furthermore, the crystal form C of cariprazine mono-1-hydroxy-2-naphthoate has an X-ray powder diffraction pattern substantially as shown in FIG. 7.

Preferably, the crystal form C of cariprazine mono-1-hydroxy-2-naphthoate has a differential scanning calorimetry pattern substantially as shown in FIG. 8.

Preferably, the crystal form C of cariprazine mono-1-hydroxy-2-naphthoate has a melting point of about 103.4 °C.

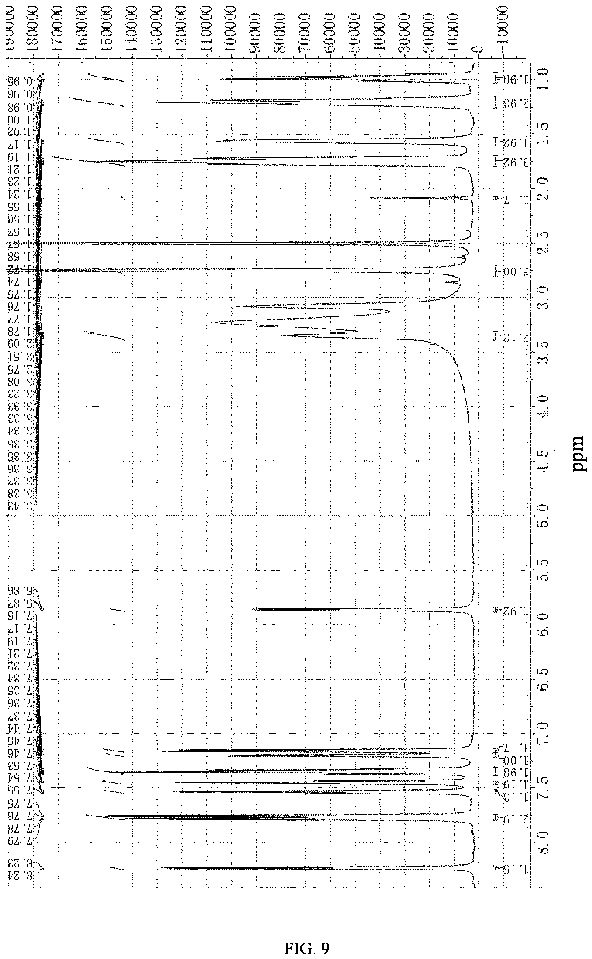

According to an embodiment of the present disclosure, in the crystal form C of cariprazine mono-1-hydroxy-2-naphthoate, cariprazine and 1-hydroxy-2-naphthoic acid form the salt in a molar ratio of 1:1, and a ¹H-NMR spectrum thereof is substantially as shown in FIG. 9.

According to an embodiment of the present disclosure, a preparation method for the crystal form C of cariprazine mono-1-hydroxy-2-naphthoate comprises the following steps:
(c1) mixing cariprazine and 1-hydroxy-2-naphthoic acid with a solvent according to a molar ratio of 1:1; and
(c2) stirring at 25-70 °C to obtain the crystal form C of cariprazine mono-1-hydroxy-2-naphthoate.

According to an embodiment of the present disclosure, the solvent is a mixed system of methanol, ethanol or acetone and water. Preferably, methanol, ethanol or acetone and water may be in a volume ratio of 2:1 to 1:2, such as 1.5:1.

Preferably, cariprazine and the total volume of the solvent system are in a mass-to-volume ratio of 1 g:(5-30) mL, such as 1 g:(5-20) mL, e.g., 1 g:10 mL.

According to an embodiment of the present disclosure, the cariprazine 1-hydroxy-2-naphthoate is cariprazine bis-1-hydroxy-2-naphthoate represented by formula (II) described above, wherein cariprazine and 1-hydroxy-2-naphthoic acid are in a molar ratio of 1:2.

According to an embodiment of the present disclosure, the cariprazine bis-1-hydroxy-2-naphthoate may be in a solid form, such as an amorphous or crystal form.

According to an embodiment of the present disclosure, the cariprazine bis-1-hydroxy-2-naphthoate is crystal form A of cariprazine bis-1-hydroxy-2-naphthoate, wherein the crystal form A of cariprazine bis-1-hydroxy-2-naphthoate has an X-ray powder diffraction pattern comprising characteristic peaks at 20 values of 4.7°±0.2°, 9.4°±0.2°, 20.8°±0.2°, etc.

Further, the crystal form A of cariprazine bis-1-hydroxy-2-naphthoate has an X-ray powder diffraction pattern comprising characteristic peaks at 20 values of 4.7°±0.2°, 9.4°±0.2°, 12.7°±0.2°, 20.1°±0.2°, 20.8°±0.2°, 27.4°±0.2°, etc.

Further, the crystal form A of cariprazine bis-1-hydroxy-2-naphthoate has an X-ray powder diffraction pattern comprising characteristic peaks at 20 values of 4.7°±0.2°, 9.4°±0.2°, 12.7°±0.2°, 17.1°±0.2°, 19.1°±0.2°, 20.1°±0.2°, 20.8°±0.2°, 27.4°±0.2°, etc.

Furthermore, according to an embodiment of the present disclosure, the crystal form A of cariprazine bis-1-hydroxy-2-naphthoate has an X-ray powder diffraction pattern comprising characteristic peaks at 20 values of 4.7°±0.2°, 8.8°±0.2°, 9.4°±0.2°, 12.7°±0.2°, 16.5°±0.2°, 17.1°±0.2°, 18.4°±0.2°, 19.1°±0.2°, 19.9°±0.2°, 20.1°±0.2°, 20.8°±0.2°, 21.1°±0.2°, 21.9°±0.2°, 24.1°±0.2°, 24.8°±0.2°, 25.6°±0.2°, 27.4°±0.2°, etc.

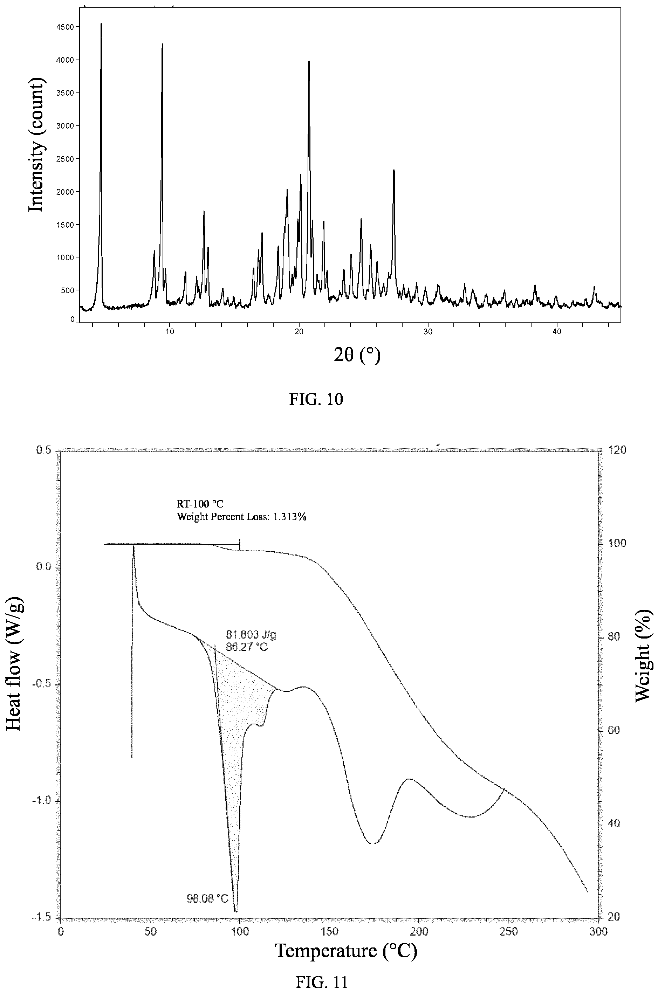

Furthermore, the crystal form A of cariprazine bis-1-hydroxy-2-naphthoate has an X-ray powder diffraction pattern substantially as shown in FIG. 10.

Preferably, the crystal form A of cariprazine bis-1-hydroxy-2-naphthoate has a differential scanning calorimetry pattern substantially as shown in FIG. 11.

Preferably, the crystal form A of cariprazine bis-1-hydroxy-2-naphthoate has a melting point of about 86.3 °C.

Preferably, the crystal form A of cariprazine bis-1-hydroxy-2-naphthoate has a thermogravimetric analysis pattern substantially as shown in FIG. 11, indicating a weight loss of about 1.31% before 100 °C.

Preferably, the crystal form A of cariprazine bis-1-hydroxy-2-naphthoate is cariprazine bis-1-hydroxy-2-naphthoate hydrate.

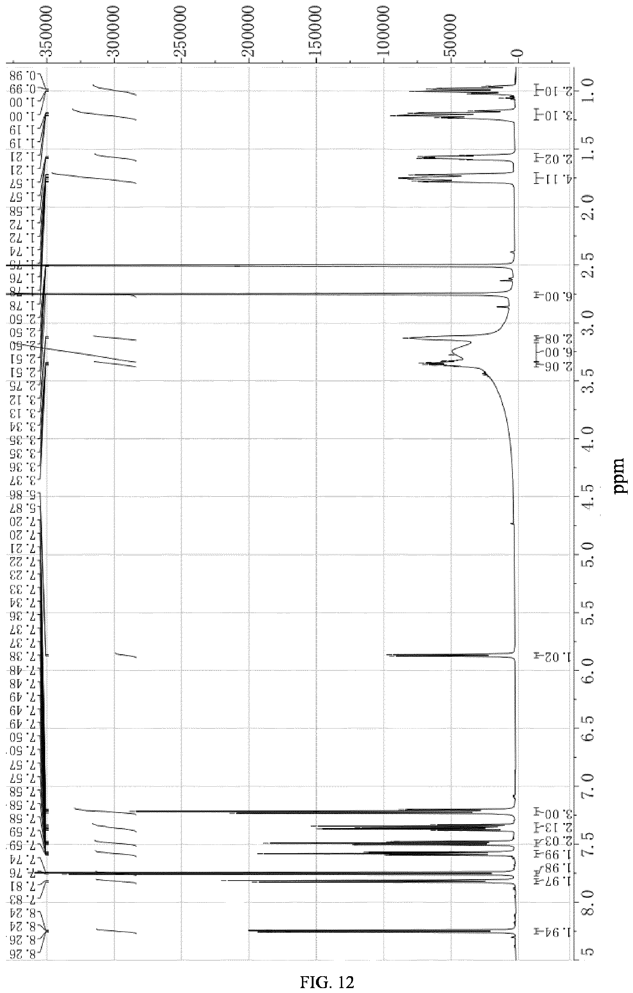

Preferably, a ¹H-NMR spectrum of the the crystal form A of cariprazine bis-1-hydroxy-2-naphthoate is substantially as shown in FIG. 12, indicating that cariprazine and 1-hydroxy-2-naphthoic acid form the salt in a molar ratio of 1:2.

According to an embodiment of the present disclosure, a preparation method for the crystal form A of cariprazine bis-1-hydroxy-2-naphthoate comprises the following steps:
adding cariprazine and 1-hydroxy-2-naphthoic acid to a solvent according to a molar ratio of 1:2 and stirring at 25-70 °C to obtain the crystal form A of cariprazine bis-1-hydroxy-2-naphthoate. The solvent is a mixed system of methanol, ethanol or acetone and water. Preferably, methanol, ethanol or acetone and water may be in a volume ratio of 2:1 to 1:2, such as 1:1.

Preferably, cariprazine and the total volume of the solvent system are in a mass-to-volume ratio of 1 g:(5-40) mL, such as 1 g:(10-30) mL, e.g., 1 g:20 mL.

According to an embodiment of the present disclosure, the cariprazine embonate has a structure represented by formula (III) shown below:

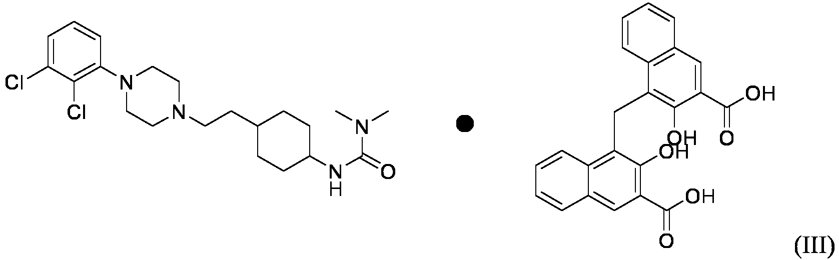

According to an embodiment of the present disclosure, cariprazine and embonic acid are in a molar ratio of 1:1 in the cariprazine embonate.

According to an embodiment of the present disclosure, the cariprazine embonate is crystal form H of cariprazine embonate, and the crystal form H of cariprazine embonate has an X-ray powder diffraction pattern comprising characteristic peaks at 20 values of 7.3°±0.2°, 8.8°±0.2°, 9.4°±0.2°, 14.6°±0.2°, 17.4°±0.2°, 18.1°±0.2°, 18.6°±0.2°, 19.2°±0.2°, 22.9°±0.2°, etc.

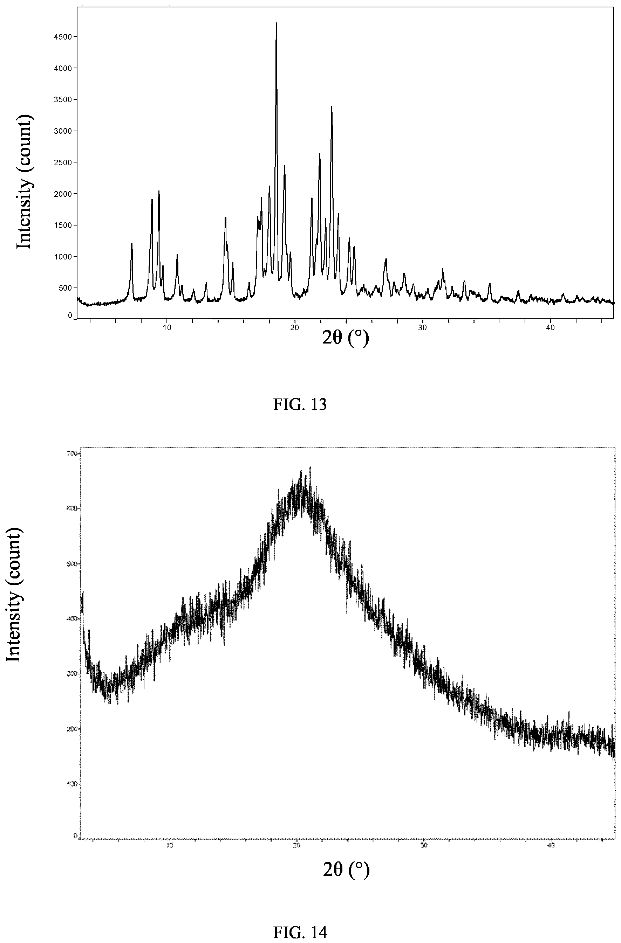

Furthermore, the crystal form H of cariprazine embonate has an X-ray powder diffraction pattern substantially as shown in FIG. 13.

According to an embodiment of the present disclosure, the cariprazine embonate is an amorphous form, which preferably has an X-ray powder diffraction pattern substantially as shown in FIG. 14.

According to an embodiment of the present disclosure, the solid form includes a crystal or non-crystal (amorphous) form of a pharmaceutically acceptable salt of cariprazine, which may be an anhydrate, a hydrate or a solvate. When present, the salt and water molecules or solvent molecules in its solid form may be in a molar ratio within a range of 1:(0.5-1:5) (e.g., 1:0.5, 1:1, 1:1.5, 1:2, 1:2.5, or 1:3), and this molar ratio may also be detected by methods well known to those skilled in the art.

The present disclosure further provides a combination of pharmaceutically acceptable salts of cariprazine, which comprises at least one, e.g., two or three, of the pharmaceutically acceptable salts of cariprazine or solid forms thereof described above, and other pharmaceutically acceptable salts of cariprazine, or other forms of cariprazine mono-1-hydroxy-2-naphthoate, cariprazine bis-1-hydroxy-2-naphthoate or cariprazine embonate, which are optionally present. According to an embodiment of the present disclosure, based on the total weight of the pharmaceutically acceptable salts of cariprazine in the combination, the content of the crystal forms of cariprazine mono-1-hydroxy-2-naphthoate or cariprazine bis-1-hydroxy-2-naphthoate described above in percentages by weight is greater than the content of the other salt forms or crystal forms of cariprazine.

For example, based on the total weight of the pharmaceutically acceptable salts of cariprazine in the combination, the content of the crystal forms of cariprazine mono-1-hydroxy-2-naphthoate or cariprazine bis-1-hydroxy-2-naphthoate described above in percentages by weight is greater than 80%, preferably greater than 90%, and more preferably greater than 95% or 99%.

The present disclosure further provides a pharmaceutical composition comprising the pharmaceutically acceptable salt of cariprazine described above, preferably comprising one of cariprazine mono-1-hydroxy-2-naphthoate, cariprazine bis-1-hydroxy-2-naphthoate, cariprazine embonate or crystal forms thereof.

The present disclosure further provides a pharmaceutical composition of cariprazine, which comprises solid particles of cariprazine that have particle sizes Dv(10) of ≤ 30 microns, Dv(50) of ≤ 50 microns and Dv(90) of ≤ 100 microns, preferably ≤ 50 microns.

The solid particles of cariprazine may be selected from one of the cariprazine mono-1-hydroxy-2-naphthoate, cariprazine bis-1-hydroxy-2-naphthoate, cariprazine embonate or crystal forms thereof described above.

According to an embodiment of the present disclosure, the pharmaceutically acceptable salt of cariprazine includes, but is not limited to, cariprazine mono-1-hydroxy-2-naphthoate, cariprazine bis-1-hydroxy-2-naphthoate and cariprazine embonate.

According to an embodiment of the present disclosure, the solid particles of cariprazine may be in amorphous or crystal form.

According to an embodiment of the present disclosure, the pharmaceutical composition of cariprazine may further comprise an auxiliary material, which may be selected from one or more of a suspending agent, a wetting agent, a tonicity adjusting agent, a solvent and a buffer. According to an embodiment of the present disclosure, the suspending agent is at a concentration in the range of 0 mg/mL to 10 mg/mL, preferably 3.5 mg/mL to 7.5 mg/mL, for example, 5.0 mg/mL or 7.5 mg/mL.

According to an embodiment of the present disclosure, the suspending agent is selected from one or more of sodium carboxymethylcellulose, methylcellulose and polyvinylpyrrolidone, preferably sodium carboxymethylcellulose.

According to an embodiment of the present disclosure, the wetting agent is at a concentration in the range of 0.2 mg/mL to 10 mg/mL, preferably 1 mg/mL to 5 mg/mL, for example, 1 mg/mL, 1.5 mg/mL, 2.0 mg/mL, 2.5 mg/mL, 3.0 mg/mL, 3.5 mg/mL, 4.0 mg/mL, 4.5 mg/mL or 5.0 mg/mL.

According to an embodiment of the present disclosure, the wetting agent is selected from one or more of tween 20, tween 80 and poloxamer 188, preferably poloxamer 188.

According to an embodiment of the present disclosure, the tonicity adjusting agent is at a concentration in the range of 20 mg/mL to 30 mg/mL, preferably 23 mg/mL to 26 mg/mL, for example, 23 mg/mL, 24.7 mg/mL or 26 mg/mL.

According to an embodiment of the present disclosure, the tonicity adjusting agent is selected from one or more of sodium chloride, mannitol and sucrose.

According to an embodiment of the present disclosure, the stabilizer is at a concentration in the range of 0 mg/mL to 30 mg/mL, preferably 1 mg/mL to 10 mg/mL, for example, 1 mg/mL, 3 mg/mL, 5 mg/mL or 7.0 mg/mL.

According to an embodiment of the present disclosure, the buffer is selected from one or more of phosphoric acid, phosphate, citric acid, sodium citrate, hydrochloric acid and sodium hydroxide.

According to an embodiment of the present disclosure, the solvent is water, for example, water for injection.

As an example, the pharmaceutical composition of cariprazine may comprise the following components:
(a) cariprazine 1-hydroxy-2-naphthoate;
(b) sodium carboxymethylcellulose or PVP K30;
(c) tween 20 or poloxamer 188;
(d) disodium hydrogen phosphate;
(e) sodium dihydrogen phosphate; and
(f) mannitol;
and, optionally, the pharmaceutical composition of cariprazine may comprise a pH regulator, such as sodium hydroxide or hydrochloric acid.

According to an embodiment of the present disclosure, the pharmaceutical composition of cariprazine may have a pH of 4.0-9.0, such as 4.0, 4.5, 5.0, 5.5, 6.0, 6.5, 7.0, 7.5, 8.0, 8.5 or 9.0. According to an embodiment of the present disclosure, the pharmaceutical composition of cariprazine is an injection, such as a long-acting injection.

According to an embodiment of the present disclosure, the pharmaceutical composition of cariprazine is a suspension or a suspension agent, preferably an aqueous suspension or an aqueous suspension agent.

According to an embodiment of the present disclosure, in the pharmaceutical composition or injection of cariprazine, the solid particles of cariprazine are at a concentration of no less than 30 mg/mL, such as 30-120 mg/mL, preferably 90 mg/mL (w/v).

According to an embodiment of the present disclosure, a preparation method for the pharmaceutical composition of cariprazine comprises mixing the components.

Preferably, the preparation method for the pharmaceutical composition of cariprazine comprises the following steps:
(1) optionally, dissolving a suspending agent, a wetting agent, a buffer, and a tonicity adjusting agent in a solvent;
(2) adding the solid particles of cariprazine to obtain an aqueous suspension of coarse particles;
(3) optionally, grinding the aqueous suspension of coarse particles described above with a ball mill to obtain a suspension of fine particles; and
(4) optionally, adding the suspending agent to the suspension of fine particles described above, mixing the suspension homogeneously, adjusting the pH to 4.0-9.0 with sodium hydroxide or hydrochloric acid optionally, and bringing the suspension to volume to obtain an aqueous suspension.

According to an embodiment of the preparation method of the present disclosure, in step (1), the suspending agent, wetting agent, buffer and tonicity adjusting agent may be sequentially dissolved in the solvent, for example, in water for injection.

According to an embodiment of the preparation method of the present disclosure, in step (4), the pH may be adjusted to 4.0-9.0, such as 4.0, 4.5, 5.0, 5.5, 6.0, 6.5, 7.0, 7.5, 8.0, 8.5 or 9.0.

The present disclosure provides use of one of the cariprazine mono-1-hydroxy-2-naphthoate, cariprazine bis-1-hydroxy-2-naphthoate, cariprazine embonate or crystal forms thereof, the combination of pharmaceutically acceptable salts of cariprazine, or the pharmaceutical composition of cariprazine described above in the manufacture of a medicament, wherein the medicament is used for treating and/or preventing a cognitive disorder or a mental disorder, such as psychosis, bipolar disorder or acute mania.

The present disclosure further provides a method for treating and/or preventing a cognitive disorder or a mental disorder, such as psychosis, bipolar disorder or acute mania, comprising administering to a patient in need thereof one of the cariprazine mono-1-hydroxy-2-naphthoate, cariprazine bis-1 -hydroxy-2-naphthoate, cariprazine embonate or crystal forms thereof described above, or the pharmaceutical composition of cariprazine.

According to an embodiment of the present disclosure, the "Dv(10)", "Dv(50)", "Dv(90)" and "Dv(100)" refer to volume-weighted particle diameters, wherein cumulative 10 v/v%, 50 v/v%, 90 v/v% or 100 v/v% of the particles have equal or smaller diameters when measured. For example, if the Dv(50) of a population of particles is about 25 microns, then 50% by volume of the particles have a diameter of less than or equal to about 25 microns.

The preferred conditions described above may be combined by those skilled in the art to obtain preferred embodiments of the present disclosure without departing from the spirit of the present disclosure.

The reagents and starting materials used in the present disclosure are commercially available. According to an embodiment of the present disclosure, the room temperature refers to an ambient temperature of 10-35 °C.

### Beneficial Effects

After extensive research, screening and trials, the inventors find that the salts of cariprazine and the solid forms thereof provided by the present disclosure, especially cariprazine 1-hydroxy-2-naphthoate that is one of the very slightly soluble salts of cariprazine, have the advantages of long-acting sustained-release preparations and can be developed into injectable suspensions while the risk of dissociation of the existing salt forms such as cariprazine hydrochloride and the like or the lack of a sustained-release effect can be overcome, and the long-acting administration can be achieved, greatly improving the compliance of patients and the bioavailability and medication safety of medicaments.

The salts of cariprazine and the pharmaceutical composition thereof of the present disclosure have the characteristics of sustained release, high bioavailability, good stability in solution, small-volume administration, etc., and after one administration, the release of cariprazine may last at least three weeks or longer. Therefore, the salts of cariprazine and the pharmaceutical composition thereof of the present disclosure have good marketing prospects.

### BRIEF DESCRIPTION OF THE DRAWINGS

FIG. 1 is an XRPD pattern of crystal form A of cariprazine mono-1-hydroxy-2-naphthoate dihydrate;
FIG. 2 is a DSC/TGA profile of crystal form A of cariprazine mono-1-hydroxy-2-naphthoate dihydrate;
FIG. 3 is a ¹H-NMR spectrum of crystal form A of cariprazine mono-1-hydroxy-2-naphthoate dihydrate;
FIG. 4 is an XRPD pattern of crystal form B of cariprazine mono-1-hydroxy-2-naphthoate;
FIG. 5 is a DSC profile of crystal form B of cariprazine mono-1-hydroxy-2-naphthoate;
FIG. 6 is a ¹H-NMR spectrum of crystal form B of cariprazine mono-1-hydroxy-2-naphthoate;
FIG. 7 is an XRPD pattern of crystal form C of cariprazine mono-1-hydroxy-2-naphthoate;
FIG. 8 is a DSC profile of crystal form C of cariprazine mono-1-hydroxy-2-naphthoate;
FIG. 9 is a ¹H-NMR spectrum of crystal form C of cariprazine bis-1-hydroxy-2-naphthoate;
FIG. 10 is an XRPD pattern of crystal form A of cariprazine bis-1-hydroxy-2-naphthoate hydrate;
FIG. 11 is a DSC/TGA profile of crystal form A of cariprazine bis-1-hydroxy-2-naphthoate hydrate;
FIG. 12 is a ¹H-NMR spectrum of crystal form A of cariprazine bis-1-hydroxy-2-naphthoate hydrate;
FIG. 13 is an XRPD pattern of crystal form H of cariprazine embonate;
FIG 14 is an XRPD pattern of amorphous cariprazine embonate;
FIG. 15 is an XRPD pattern of crystal form I of cariprazine hydrochloride;
FIG. 16 is an overlay of XRPD patterns in the cariprazine hydrochloride dissociation study at pH 7.4 in Example 44, wherein A indicates cariprazine free base; B indicates crystal form I of cariprazine hydrochloride; and C indicates a sample of the crystal form I of cariprazine hydrochloride that has been shaken in a medium with the pH of 7.4;
FIG. 17 is a graph showing a relationship between the mean plasma concentration of cariprazine and time in a rat, for an oral liquid sample of the preparation of Example 48 of the present disclosure;
FIG. 18 is a graph showing relationships between the mean plasma concentration of cariprazine and time in rats, for injection samples of the preparations of Examples 45-47 of the present disclosure, wherein ▪ indicates crystal form A of cariprazine mono-1-hydroxy-2-naphthoate dihydrate; ▲ indicates crystal form A of cariprazine bis-1-hydroxy-2-naphthoate hydrate; and ◆ indicates amorphous cariprazine embonate;
FIG. 19 is a partially enlarged view of FIG. 18 (showing the relationships between the mean plasma concentration of cariprazine and the time from 0 to 24 h, wherein ▪ indicates crystal form A of cariprazine mono-1-hydroxy-2-naphthoate dihydrate; ▲ indicates crystal form A of cariprazine bis-1-hydroxy-2-naphthoate hydrate; and ◆ indicates amorphous cariprazine embonate;
FIG. 20 is a graph illustrating relationships between the mean plasma concentration of cariprazine and time in rats of different sexes, for an injection sample of the preparation of Examples 42 of the present disclosure, wherein ▪ is a graph showing the relationship of crystal form A of cariprazine mono-1-hydroxy-2-naphthoate dihydrate in male rats; and is a graph showing the relationship of crystal form A of cariprazine mono-1-hydroxy-2-naphthoate dihydrate in female rats;
FIG. 21 is a partially enlarged view of FIG. 18 (showing the relationships between the mean plasma concentration of cariprazine and the time from 0 to 24 h, wherein ▪ is a graph showing the relationship of crystal form A of cariprazine mono-1-hydroxy-2-naphthoate dihydrate in male rats; and ▲ is a graph showing the relationship of crystal form A of cariprazine mono-1-hydroxy-2-naphthoate dihydrate in female rats;
FIG. 22 is an X-ray diffraction pattern of a single crystal of crystal form A of cariprazine mono-1-hydroxy-2-naphthoate dihydrate;
FIG. 23 is an ellipsoid plot of the three-dimensional structure of the single crystal molecule of cariprazine mono-1-hydroxy-2-naphthoate dihydrate;
FIG. 24 is a unit cell packing projection diagram of the single crystal of cariprazine mono-1-hydroxy-2-naphthoate dihydrate along the b-axis.

### DETAILED DESCRIPTION

The present disclosure is further illustrated by the following examples; however, these examples should not be construed as limiting the present disclosure. Experimental procedures without specified conditions in the following examples are conducted in accordance with conventional procedures and conditions, or in accordance with the manufacturer's manual.

The salt compounds of the examples were tested by nuclear magnetic resonance (¹H-NMR), X-ray powder diffraction (XRPD), differential scanning calorimetry (DSC), thermogravimetric analysis (TGA) and high performance liquid chromatography (HPLC), with test parameters as follows:
(1) The ¹H-NMR measurements were conducted on a Bruker Advance III500M nuclear magnetic resonance spectrometer at a frequency of 400 Mz with deuterated DMSO as the solvent.
(2) The DSC measurements were conducted on a sealed pan device in TA Instruments model 25: samples (about 1-2 mg) were weighed in aluminum pans and transferred to the instrument for measurement. The test parameters are as follows: the instrument was equilibrated at 40 °C and heated to 230-300 °C at a rate of 10-20 °C/min, data were collected, and the experiments were conducted in a nitrogen atmosphere.
(3) The TGA measurements were conducted on a TA Instruments model 55 device: samples (about 2-5 mg) were weighed in aluminum pans and transferred to the instrument for measurement. The test parameters are as follows: the instrument was heated to 300 °C at a rate of 10 °C/min, data were collected, and the experiments were conducted in a nitrogen atmosphere.
(4) The XRPD measurements were conducted on a Bruker model D8 Advance X-ray powder diffractometer using a round zero background single crystal silicon sample stage. CuKa rays were used as a light source in the test, and the scanning parameters are as follows: voltage: 40 kv; current: 40 mA; scanning range: 3°-45°; scanning step size: 0.02°; scanning mode:
continuous scanning.
(5) Single crystal detection method: referring to Method 1 of General Chapter 0451, *Chinese Pharmacopoeia,* Volume IV, 2020 Edition, a single crystal X-ray diffraction analysis was conducted using a Bruker model SMART APEX-II single crystal X-ray diffractometer, and the test conditions are as follows: CuKa radiation; voltage: 40 kv; current: 30 mA; scanning range: 4.31°-66.50°; scanning mode: ϕ/ω; scanning step size: 0.5°.
(6) Methods for measuring HPLC content and detecting related substances:
Content method:

| | |
|---|---|
| Instrument | High performance liquid chromatography |
| Chromatographic column | Welch Ultimate^{®} XB-C18 4.6×250 mm, 5 µm |
| Detection wavelength | 217 nm |
| Flow rate | 1.0 ml/min |
| Column temperature | 30 °C |
| Injection volume | 5 µl |
| Mobile phase A | 0.025 mol/L potassium dihydrogen phosphate solution (pH = 2.4) |
| Mobile phase B | Acetonitrile |
| Solvent | 80% methanol |

Content gradient elution program table:

| Time (min) | Mobile phase A (%) | Mobile phase B (%) |
|---|---|---|
| 0 | 60 | 40 |
| 2 | 40 | 60 |
| 5 | 15 | 85 |
| 5.1 | 60 | 40 |
| 10 | 60 | 40 |

Related substances method:

| | |
|---|---|
| Instrument | High performance liquid chromatography |
| Chromatographic column | Welch Ultimate^{®} XB-C18 4.6x250 mm, 5 µm |
| Detection wavelength | 217 nm |
| Flow rate | 1.0 ml/min |
| Column temperature | 30 °C |
| Injection volume | 10 µl |
| Mobile phase A | 0.025 mol/L potassium dihydrogen phosphate solution (pH = 2.4) |
| Mobile phase B | Acetonitrile |

| | |
|---|---|
| Solvent | 80% methanol |

Related gradient elution program table:

| Time (min) | Mobile phase A (%) | Mobile phase B (%) |
|---|---|---|
| 0 | 80 | 20 |
| 7 | 45 | 55 |
| 17 | 37 | 63 |
| 25 | 20 | 80 |
| 25.1 | 80 | 20 |
| 35 | 80 | 20 |

Unless otherwise indicated, the aqueous suspensions of the pharmaceutical composition of cariprazine in the context of the present disclosure were measured on a particle size detection instrument under the following conditions:

| | |
|---|---|
| Injector: SCF-105B | Substance: pharmaceutical composition of cariprazine |
| Refractive index of particles: 1.595 | Refractive index of dispersant: 1.333 |
| Dispersant: water | Background test time: 10 s |
| Sample test time: 10 s | Obscuration: 10-20% |
| Stirring speed: 600-2000 r/min | Analysis model: universal |
| Measurement range: 0.02-2100 µm | Particle absorption rate: 0.1 |

Measurement was performed in triplicate, and a mean value was produced.

### Example 1A: Preparation of Crystal Form A of Cariprazine Mono-1-hydroxy-2-naphthoate Dihydrate

0.5 g of cariprazine free base was taken and added to a 10 mL centrifuge tube, 4 mL of pure water solution was added, and then 1 mL of phosphoric acid solution with a concentration of 100 mL/mL was added; and the mixture was ultrasonically dissolved and filtered to a 30 mL vial to obtain solution A. 0.22 g of 1-hydroxy-2-naphthoic acid was taken and added to a 5 mL centrifuge tube, 4 mL of pure water solution was added, and then 0.936 mL of sodium hydroxide solution (50 mg/mL) was added; and the mixture was ultrasonically dissolved to obtain solution B. The solution B was slowly filtered into the solution A under stirring, and a grey-brown solid was precipitated. The mixture was stirred at room temperature overnight, filtered under vacuum, and dried under reduced pressure at 40 °C overnight to obtain the crystal form A of cariprazine mono-1-hydroxy-2-naphthoate (0.7 g, yield: 97%, HPLC purity: 99.19%).

Its X-ray powder diffraction pattern is shown in FIG. 1.

Its differential scanning calorimetry pattern is shown in FIG. 2, indicating a melting point of about 101.3 °C.

Its thermogravimetric analysis pattern is shown in FIG. 2, indicating a weight loss of about 4.82% before 100 °C due to loss of crystal water and adsorbed water, and the crystal form A is cariprazine mono-1-hydroxy-2-naphthoate dihydrate.

Its nuclear magnetic resonance spectrum is shown in FIG. 3, indicating that cariprazine and 1-hydroxy-2-naphthoic acid formed the salt in a molar ratio of 1:1.

### Example 1B: Preparation of Single Crystal of Crystal Form A of Cariprazine Mono-1-hydroxy-2-naphthoate Dihydrate

0.25 g of cariprazine free base and 0.1075 g of 1-hydroxy-2-naphthoic acid were taken, and 2.5 mL of a mixed reagent of acetone and water in a ratio of 6:4 was added. The mixture was stirred at 70 °C for 0.5 h, dissolved, and filtered. Heating was stopped, and then the mixture was left to stand overnight. The sample was sent for single crystal detection.

Referring to Method 1 of General Chapter 0451, *Chinese Pharmacopoeia,* Volume IV, 2020 Edition, the single crystal X-ray diffraction analysis was conducted using a Bruker model SMART APEX-II single crystal X-ray diffractometer, and the test conditions are as follows: CuKa radiation; voltage: 40 kv; current: 30 mA; scanning range: 4.31°-66.50°; scanning mode: ϕ/ω; scanning step size: 0.5°.

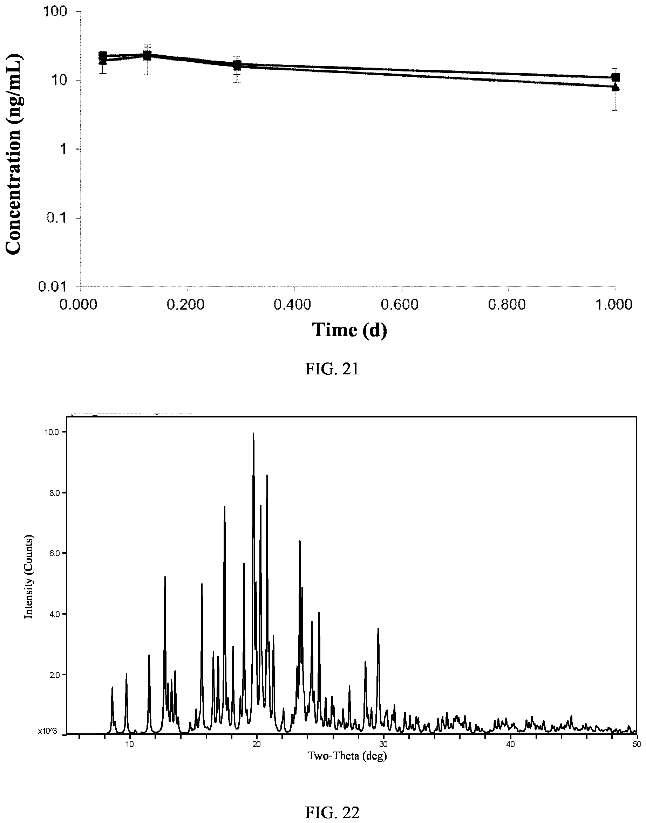

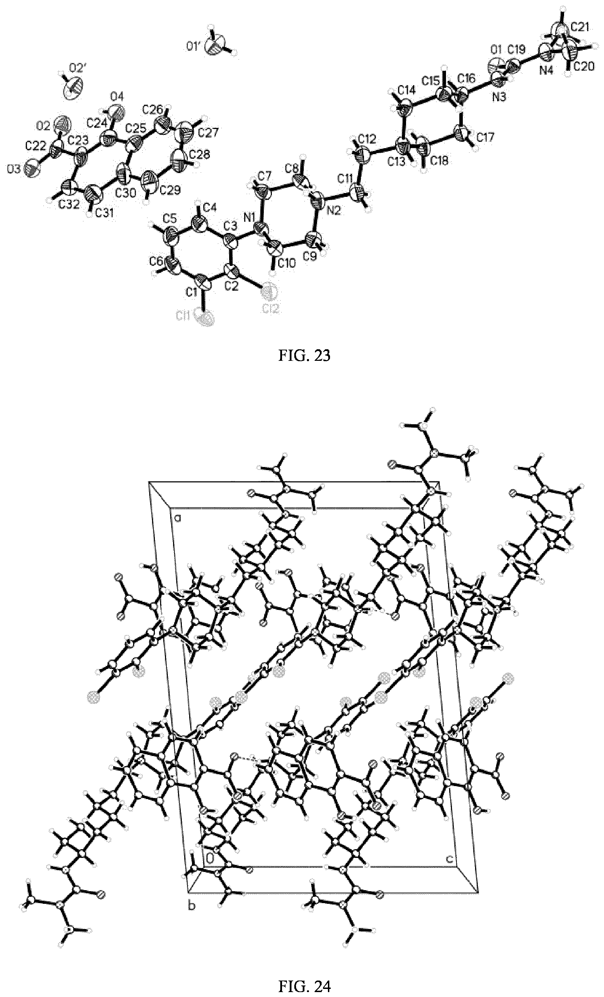

The X-ray diffraction pattern of the single crystal of the obtained sample is shown in FIG. 22, the ellipsoid plot of the three-dimensional structure of the single crystal molecule is shown in FIG. 23, and the unit cell packing projection diagram of the single crystal along the b-axis is shown in FIG. 24.

### Example 2: Preparation of Crystal Form A of Cariprazine Mono-1-hydroxy-2-naphthoate Dihydrate

100 g of cariprazine free base and 43 g of 1-hydroxy-2-naphthoic acid were taken, and 1000 mL of a mixed reagent of ethanol and water in a ratio of 1:1 was added. The mixture was stirred and dissolved at 70 °C. Heating was stopped, and then the mixture was cooled to precipitate a solid. The mixture was stirred for another 5 h, filtered under vacuum, and dried under reduced pressure at 40 °C overnight to obtain the crystal form A of cariprazine mono-1-hydroxy-2-naphthoate dihydrate (13.6 g, yield: 95%, HPLC purity: 99.59%).

This crystal form was subjected to hydrogen nuclear magnetic resonance, X-ray powder diffraction, differential scanning calorimetry and thermogravimetric analysis, and found that it was consistent with the chemical structure and crystal structure of Example 1A, had a melting point of about 103.7 °C, and had a weight loss of about 4.45% before 100 °C.

### Example 3: Preparation of Crystal Form B of Cariprazine Mono-1-hydroxy-2-naphthoate

1 g of cariprazine free base and 0.43 g of 1-hydroxy-2-naphthoic acid were taken, and 1 mL of a mixed reagent of acetone and water in a ratio of 6:4 was added. The mixture was stirred and dissolved at 70 °C, and then filtered. Heating was stopped, and then the mixture was left to stand at room temperature overnight, filtered under vacuum, and dried under reduced pressure at 60 °C for 1 h to obtain the crystal form B of cariprazine mono-1-hydroxy-2-naphthoate (1.27 g, yield: 89%).

Its X-ray powder diffraction pattern is shown in FIG. 4.

Its differential scanning calorimetry pattern is shown in FIG. 5, indicating a melting point of about 106.8 °C.

Its nuclear magnetic resonance spectrum is shown in FIG. 6, indicating that cariprazine and 1-hydroxy-2-naphthoic acid formed the salt in a molar ratio of 1:1.

### Example 4: Preparation of Crystal Form C of Cariprazine Mono-1-hydroxy-2-naphthoate

10 g of cariprazine free base and 4.3 g of 1-hydroxy-2-naphthoic acid were taken, and 100 mL of a mixed reagent of acetone and water in a ratio of 6:4 was added. The mixture was stirred and dissolved at 70 °C, and then filtered. Heating was stopped, and then the mixture was left to stand at room temperature overnight, filtered under vacuum, and dried under reduced pressure at 60 °C for 5 h to obtain the crystal form C of cariprazine mono-1-hydroxy-2-naphthoate (10.8 g, yield: 76%).

Its X-ray powder diffraction pattern is shown in FIG. 7.

Its differential scanning calorimetry pattern is shown in FIG. 8, indicating a melting point of about 103.4 °C.

Its nuclear magnetic resonance spectrum is shown in FIG. 9, indicating that cariprazine and 1-hydroxy-2-naphthoic acid formed the salt in a molar ratio of 1:1.

### Example 5: Preparation of Crystal Form A of Cariprazine Bis-1-hydroxy-2-naphthoate Hydrate

1 g of cariprazine free base and 0.85 g of 1-hydroxy-2-naphthoic acid were taken, and 20 mL of a mixed reagent of ethanol and water in a ratio of 1:1 was added. The mixture was stirred at 70 °C for 2 h. Heating was stopped, and then the mixture was stirred for another 3 h, filtered under vacuum, and dried under reduced pressure at 40 °C overnight to obtain the crystal form A of cariprazine bis-1-hydroxy-2-naphthoate (1.63 g, yield: 88%, HPLC purity: 99.44%).

Its X-ray powder diffraction pattern is shown in FIG. 10.

Its differential scanning calorimetry pattern is shown in FIG. 11, indicating a melting point of about 86.3 °C.

Its thermogravimetric analysis pattern is shown in FIG. 11, indicating a weight loss of about 1.31% before 100 °C due to loss of crystal water and adsorbed water, and the crystal form A is cariprazine bis-1-hydroxy-2-naphthoate hydrate.

Its nuclear magnetic resonance spectrum is shown in FIG. 12, indicating that cariprazine and 1-hydroxy-2-naphthoic acid formed the salt in a molar ratio of 1:2.

**Example 6: Preparation of Crystal Form H of Cariprazine Embonate**

2 g of commercially available cariprazine free base and 1.62 g of embonic acid were dissolved in 80 mL of a solvent of tetrahydrofuran and methanol (2:1) at 60 °C. The mixture was filtered, and the solvent was removed by rotary evaporation to obtain a yellow solid. 30 mL of methanol was added, and the mixture was dissolved at 60 °C. The solvent was removed by rotary evaporation, and the residue was dried to obtain a light yellow solid. 0.1 g of the light yellow solid described above was taken, and 2.5 mL of dibutyl ketone was added. The mixture was dissolved at 60 °C and filtered, and 5 volumes of n-heptane was slowly added under stirring at room temperature. The resulting mixture was stirred for 1 month and filtered to obtain the crystal form H of cariprazine embonate (0.08 g, yield: 80%).

Its X-ray powder diffraction pattern is shown in FIG. 13, indicating crystal form H of cariprazine embonate.

**Example 7: Preparation of Amorphous Cariprazine Embonate**

4000 mg (9.36 mmol) of cariprazine was dissolved in 200 mL of a phosphoric acid solution (5.4 mg/mL) to obtain solution A. 3634 mg (9.36 mmol) of embonic acid was dissolved in 100 mL of a sodium hydroxide solution (7.5 mg/mL) to obtain solution B. 100 mL of solution B was added to 200 mL of solution A over 30 min under stirring. The product was separated by filtration, washed with water and dried *in vacuo* at 40 °C for 12 h to obtain a pale yellow solid (5840 mg, yield: 76% (calculated based on free base)).

The structure and molar ratio of the cariprazine embonate of the present disclosure were confirmed by hydrogen nuclear magnetic resonance.

¹H-NMR (400MHz, DMSO-d6): δ8.38 (s, 2H), 8.16 (d, 2H), 8.80 (d, 2H), 7.39-7.13 (m, 7H), 5.86 (d, 1H), 4.76 (s, 2H), 3.40-3.32 (m, 3H), 3.22-3.18 (m, 4H), 2.75 (s, 6H), 1.76 (t, 4H), 1.63-1.57 (m, 2H), 1.25-1.16 (m, 3H), 1.04-0.96 (m, 2H).

The nuclear magnetic resonance result shows that cariprazine and embonic acid formed the salt in a molar ratio of 1:1.

The sample described above was analyzed by solid-state characterization. The XRPD pattern is shown in FIG. 14. The result indicates an amorphous cariprazine embonate.

**Example 8: Preparation of Crystal Form I of Cariprazine Hydrochloride**

Preparation of a sample of crystal form I of cariprazine hydrochloride according to the original cariprazine hydrochloride patent:
1 g of commercially available cariprazine free base was added to a 25 mL round-bottom flask. 2 mL of methanol and 8 mL of water were added. The mixture was stirred in an oil bath at 70 °C for 0.5 h. A mixed solution of 0.226 mL of concentrated hydrochloric acid and 0.35 mL of water was added. After complete dissolution, the solution was filtered while hot. Heating was stopped and the solution was naturally cooled overnight to obtain an off-white solid (0.8 g).

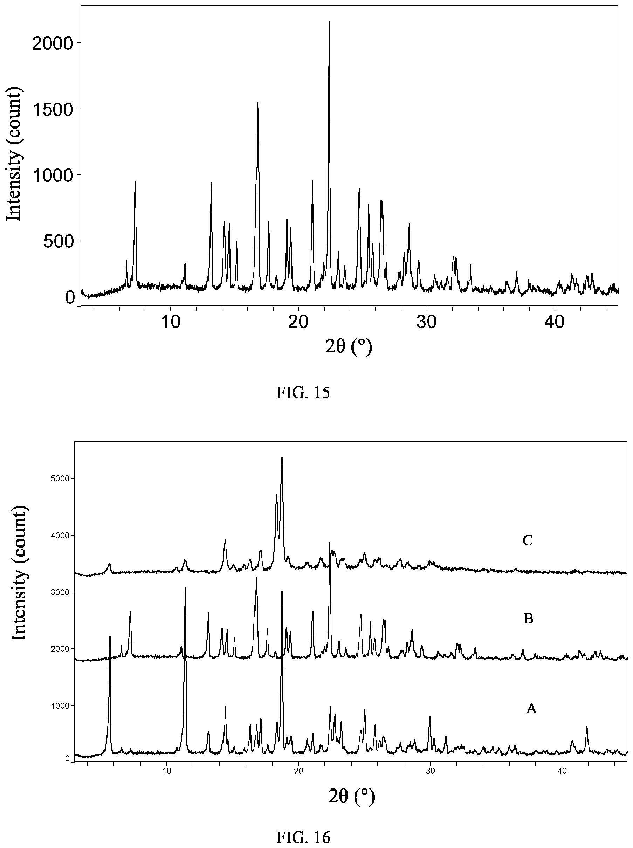

Its X-ray powder diffraction pattern is shown in FIG. 15, indicating crystal form I of cariprazine hydrochloride.

### Example 9: Related Substances and Crystal Form Stability Comparisons

The crystal form A of cariprazine mono-1-hydroxy-2-naphthoate dihydrate prepared in Example 2, the crystal form A of cariprazine bis-1-hydroxy-2-naphthoate hydrate prepared in Example 5, the amorphous cariprazine embonate prepared in Example 7 and the crystal form I of cariprazine hydrochloride prepared in Example 8 were left to stand under high-temperature (60 °C), high-humidity (25 °C/90% RH), acceleration (40 °C/75% RH) and illumination (1.2 × 10⁶ Lux · h) conditions, and samples were taken on day 0 and day 10 and tested by HPLC or XRPD.

The detection results of related substances are shown in Table 1, indicating that the changes in the related substances were all within 0.3% after the crystal form A of cariprazine mono-1-hydroxy-2-naphthoate dihydrate and the crystal form A of cariprazine bis-1-hydroxy-2-naphthoate hydrate of the present disclosure were left to stand under the high-temperature, high-humidity and acceleration conditions for 10 days, and the increase was less than that of the embonate under the high-temperature condition; similar to embonate, the impurities increased slightly under the illumination condition, suggesting that the crystal forms can still maintain good stability by adopting proper light-shielding measures.

The crystal form stability results are shown in Table 2, indicating that compared with the known crystal form I of cariprazine hydrochloride, the crystal form A of cariprazine bis-1-hydroxy-2-naphthoate hydrate of the present disclosure had more excellent crystal form stability, and the crystal form did not change and its crystallinity also did not change significantly after the crystal form A was left to stand under these conditions for 10 days. The crystal form A of cariprazine mono-1-hydroxy-2-naphthoate dihydrate of the present disclosure was relatively stable under the high-humidity, illumination and acceleration conditions, and the crystal form did not change; however, the crystal form A would change into the crystal form C of cariprazine mono-1-hydroxy-2-naphthoate under the high-temperature condition.

**Table 1. Related substances results**

| Name | Time | Changes in total related substances relative to day 0 (%) | | | |
|---|---|---|---|---|---|
| | | High temperature | High humidity | Illumination | Acceleration |
| Crystal form I of cariprazine hydrochloride | Day 0 | 0 | | | |
| | Day 10 | -0.04 | -0.03 | 0.02 | 0 |
| Amorphous cariprazine embonate | Day 0 | 0 | | | |
| | Day 10 | 0.83 | -0.05 | 0.31 | 0.11 |
| Crystal form A of cariprazine mono-1-hydroxy-2-naphthoate dihydrate | Day 0 | 0 | | | |
| | Day 10 | 0.08 | 0.04 | 0.59 | 0 |
| Crystal form A of cariprazine bis-1-hydroxy-2-naphthoate hydrate | Day 0 | 0 | | | |
| | Day 10 | 0.22 | 0.23 | 0.26 | 0.33 |

**Table 2. Crystal form stability results**

| Name | Time | Crystal form results | | | |
|---|---|---|---|---|---|
| | | High temperature | High humidity | Illumination | Acceleration |
| Crystal form I of cariprazine hydrochloride | Day 10 | Crystal form did not change | Crystal form did not change | Crystal form did not change | Crystallinity decreased |
| Crystal form A of cariprazine mono-1-hydroxy-2-naphthoate dihydrate | Day 10 | Crystal form C | Crystal form did not change | Crystal form did not change | Crystal form did not change |
| Crystal form A of cariprazine bis-1-hydroxy-2-naphthoate hydrate | Day 10 | Crystal form did not change | Crystal form did not change | Crystal form did not change | Crystal form did not change |

### Example 10: Comparison of Solubility with Different pH Values

The crystal form A of cariprazine mono-1-hydroxy-2-naphthoate dihydrate prepared in Example 1A, the crystal form C of cariprazine hemimono-1-hydroxy-2-naphthoate prepared in Example 4, the crystal form A of cariprazine bis-1-hydroxy-2-naphthoate hydrate prepared in Example 5, the amorphous cariprazine embonate prepared in Example 7, the crystal form I of cariprazine hydrochloride prepared in Example 8 and the cariprazine free base (commercially available) were added to the following media with different pH values. The mixtures were shaken at 37 °C for 24 h and filtered through a 0.45 µm aqueous-phase filter membranes. The filtrates were collected for solubility determination by high performance liquid chromatography. pH 3, pH 4, pH5 and pH 6 represent acetate buffer solutions, and pH 7, pH 7.4, pH 8 and pH 9 represent phosphate buffer solutions.

The solubility test results are shown in Table 3, indicating that the solubilities of cariprazine mono-1-hydroxy-2-naphthoate and the crystal forms thereof as well as cariprazine bis-1-hydroxy-2-naphthoate prepared in the present disclosure were all relatively low in the range of pH 3-9, the solubilities thereof were significantly lower than that of hydrochloride in the range of pH 3-6, and the solubilities thereof were significantly lower than that of embonate in the range of pH 7-9. The relatively low solubility allows the release rates of the salt forms and the crystal forms depend on the pH to the minimum extent, thereby avoiding the influence of pH environments in different areas on their drug release rates in the body, avoiding burst releases or excessive plasma concentrations in local areas in the body, and reducing the difference in drug release between individuals. In addition, as cariprazine 1-hydroxy-2-naphthoate has relatively good crystal form stability, it is suitable for use in long-acting preparations and allows reduced frequency of administration and thus improved patient compliance, thereby having a good marketing prospect.

**Table 3. Solubility results with different pH values**

| Compound | Solubility (µg/mL) | | | | | | | | |
|---|---|---|---|---|---|---|---|---|---|
| | pH 3 | pH 4 | pH 5 | pH 6 | pH 7 | pH 7.4 | pH 8 | pH 9 | Water |
| Cariprazine (commercially available) | 1363.4 | / | 242.68 | / | 3.1 8 | / | 0.4 | 1.41 | 179.94 |
| Cariprazine hydrochloride I (Example 8) | 12302.4 5 | 9765.9 | 1081.3 5 | 243.79 | 9.4 1 | 2.82 | 0.91 | 0.35 | 10935.1 4 |
| Amorphous cariprazine embonate (Example 7) | 151.2 | 19.1 | 17.7 | 19.0 | 18. 8 | 9.9 | 4.6 | 4.2 | 10.4 |
| Crystal form A of cariprazine mono-1-hydroxy-2-naphthoate dihydrate (Example 1) | 166.74 | 72.47 | 72.69 | 41.95 | 5.0 2 | 1.51 | 0.62 | 0.45 | 60.01 |
| Crystal form C of cariprazine mono-1-hydroxy-2-naphthoate (Example 4) | 102.65 | 82.64 | 81.47 | 36.38 | 4.7 9 | 1.53 | 0.51 | 0.31 | 65.28 |
| Crystal form A of cariprazine bis-1-hydroxy-2-naphthoate hydrate (Example 5) | 36.75 | 65.87 | 20.69 | 15.24 | 6.1 3 | 3.00 | 1.14 | 0.94 | 55.20 |

### Examples 11-14: Aqueous Suspensions with Different Amounts of Cariprazine Mono-1-hydroxy-2-naphthoate

| Component | Amount (mg) | | | |
|---|---|---|---|---|
| | Example 11 | Example 12 | Example 13 | Example 14 |
| Crystal form C of cariprazine mono-1-hydroxy-2-naphthoate (Example 4) | 43 | 65 | 86 | 108 |
| Tween 20 | 1.0 | | | |
| Disodium hydrogen phosphate | 4.5 | | | |
| Sodium dihydrogen phosphate | 0.9 | | | |
| Mannitol | 24.7 | | | |
| Sodium carboxymethylcellulose | 7.5 | | | |
| Aqueous sodium hydroxide solution (1 N) | Proper amount | | | |
| Aqueous hydrochloric acid solution (1 N) | Proper amount | | | |
| Water for injection | qs1.0 mL | | | |

Preparation:
(1) The formula amounts of tween 20, disodium hydrogen phosphate, sodium dihydrogen phosphate, mannitol and sodium carboxymethylcellulose were measured out, added to water for injection that was about 60% of the total amount of the preparation, and dissolved and dispersed by stirring.
(2) The formula amount of the crystal form C of cariprazine mono-1-hydroxy-2-naphthoate was added, and optionally, the pH was adjusted to 4.0-9.0 with sodium hydroxide or hydrochloric acid. The mixtures were brought to volume and completely dispersed by stirring to obtain suspensions of Examples 11-14.

The syringeability, suspendibility, settling ratio and redispersibility of the formula samples prepared in Examples 11-14 were investigated, and the investigation revealed that the suspension samples described above could all pass through 0.45 × 15 mm syringe needles and had good suspendibility, settling ratios greater than 0.9 for at least 10 min and good redispersibility (the suspension samples could be rapidly dispersed by shaking after settling).

### Examples 15-19: Aqueous Suspensions of Cariprazine Mono-1-hydroxy-2-naphthoate with Different Amounts of Tween

| Component | Amount (mg) | | | | |
|---|---|---|---|---|---|
| | Example 15 | Example 16 | Example 17 | Example 18 | Example 19 |
| Crystal form C of cariprazine mono-1-hydroxy-2-naphthoate | 130 | | | | |
| (Example 4) | | | | | |
| Tween 20 | 1.0 | 2.5 | 5.0 | 7.5 | 10.0 |
| Disodium hydrogen phosphate | 4.5 | | | | |
| Sodium dihydrogen phosphate | 0.9 | | | | |
| Mannitol | 24.7 | | | | |
| Sodium carboxymethylcellulose | 7.5 | | | | |
| Aqueous sodium hydroxide solution (1 N) | Proper amount | | | | |
| Aqueous hydrochloric acid solution (1 N) | Proper amount | | | | |
| Water for injection | qs1.0 mL | | | | |

### Preparation:

(1) The formula amounts of tween 20, disodium hydrogen phosphate, sodium dihydrogen phosphate, mannitol and sodium carboxymethylcellulose were measured out, added to water for injection that was about 60% of the total amount of the preparation, and dissolved and dispersed by stirring.
(2) The formula amount of the crystal form C of cariprazine mono-1-hydroxy-2-naphthoate was added, and optionally, the pH was adjusted to 4.0-9.0 with sodium hydroxide or hydrochloric acid. The mixtures were brought to volume and completely dispersed by stirring to obtain suspensions of Examples 15-19.

The syringeability, suspendibility, wettability, settling ratio and redispersibility of the formula samples prepared in Examples 15-19 were investigated, and the investigation revealed that the suspension samples described above could all pass through 0.45 × 15 mm syringe needles and had good suspendibility, the samples of Examples 15-19 had settling ratios greater than 0.9 for at least 10 min and good redispersibility (the suspension samples could be rapidly dispersed by shaking after settling), and the samples of Examples 16-19 had good wettability due to less adhesion of drugs to the walls of the vials.

### Examples 20-25: Aqueous Suspensions of Cariprazine 1-hydroxy-2-naphthoate with Different Amounts of Poloxamer

| Component | Amount (mg) | | | | | |
|---|---|---|---|---|---|---|
| | Exampl e 20 | Example 21 | Example 22 | Example 23 | Example 24 | Example 25 |
| Crystal form A of cariprazine mono-1-hydroxy-2-naphthoate dihydrate (Example 2) | 137 | | | - | - | - |
| Crystal form A of cariprazine bis-1-hydroxy-2-naphthoate hydrate (Example 5) | - | - | - | 170 | | |
| Poloxamer 188 | 0.5 | 1.0 | 1.5 | 0.5 | 1.0 | 1.5 |
| Disodium hydrogen phosphate | 4.5 | | | | | |
| Sodium dihydrogen phosphate | 0.9 | | | | | |
| Mannitol | 24.7 | | | | | |
| Sodium carboxymethylcellulose | 7.5 | | | | | |
| Aqueous sodium hydroxide solution (1 N) | Proper amount | | | | | |
| Aqueous hydrochloric acid solution (1 N) | Proper amount | | | | | |
| Water for injection | qs1.0 mL | | | | | |

### Preparation:

(1) The formula amounts of poloxamer 188, disodium hydrogen phosphate, sodium dihydrogen phosphate, mannitol and sodium carboxymethylcellulose were measured out, added to water for injection that was about 60% of the total amount of the preparation, and dissolved and dispersed by stirring.
(2) The formula amount of the crystal form A of cariprazine mono-1-hydroxy-2-naphthoate dihydrate or crystal form A of cariprazine bis-1-hydroxy-2-naphthoate hydrate was added, and optionally, the pH was adjusted to 4.0-9.0 with sodium hydroxide or hydrochloric acid. The mixtures were brought to volume and completely dispersed by stirring to obtain suspensions of Examples 20-25.

The syringeability, suspendibility, wettability, settling ratio and redispersibility of the formula samples prepared in Examples 20-25 were investigated, and the investigation revealed that the suspension samples described above could all pass through 0.45 × 15 mm syringe needles and had good suspendibility and good wettability due to less adhesion of drugs to the walls of the vials, and the samples of Examples 20-25 had settling ratios greater than 0.9 for at least 10 min and good redispersibility (the suspension samples could be rapidly dispersed by shaking after settling).

### Examples 26-31: Aqueous Suspensions of Cariprazine Mono-1-hydroxy-2-naphthoate with Different Amounts of Sodium Carboxymethylcellulose

| Component | Amount (mg) | | | | | |
|---|---|---|---|---|---|---|
| | Example 26 | Example 27 | Example 28 | Example 29 | Example 30 | Example 31 |
| Crystal form C of cariprazine mono-1-hydroxy-2-naphthoate (Example 4) | 130 | | | | | |
| Tween 20 | 2.5 | | | | | |
| Disodium hydrogen phosphate | 4.5 | | | | | |
| Sodium dihydrogen phosphate | 0.9 | | | | | |
| Mannitol | 24.7 | | | | | |
| Sodium carboxymethylcellulose | 1.5 | 2.5 | 3.5 | 5.0 | 7.5 | 10.0 |
| Aqueous sodium hydroxide solution (1 N) | Proper amount | | | | | |
| Aqueous hydrochloric acid solution (1 N) | Proper amount | | | | | |
| Water for injection | qs1.0 mL | | | | | |

### Preparation:

(1) The formula amounts of tween 20, disodium hydrogen phosphate, sodium dihydrogen phosphate, mannitol and sodium carboxymethylcellulose were measured out, added to water for injection that was about 60% of the total amount of the preparation, and dissolved and dispersed by stirring.
(2) The formula amount of the crystal form C of cariprazine mono-1-hydroxy-2-naphthoate was added, and optionally, the pH was adjusted to 4.0-9.0 with sodium hydroxide or hydrochloric acid. The mixtures were brought to volume and completely dispersed by stirring to obtain suspensions of Examples 26-31.

The syringeability, suspendibility, settling ratio and redispersibility of the formula samples prepared in Examples 26-31 were investigated, and the investigation revealed that the suspension samples described above could all pass through 0.45 × 15 mm syringe needles and had good suspendibility, and the samples of Examples 26-31 had settling ratios greater than 0.9 for at least 10 min and good redispersibility (the suspension samples could be rapidly dispersed by shaking after settling).

### Examples 32 and 33: Aqueous Suspensions of Cariprazine Mono-1-hydroxy-2-naphthoate with Different Amounts of PVP K30

| Component | Amount (mg) | |
|---|---|---|
| | Example 32 | Example 33 |
| Crystal form A of cariprazine mono-1-hydroxy-2-naphthoate dihydrate (Example 2) | 137 | |
| Tween 20 | 2.5 | |
| Disodium hydrogen phosphate | 4.5 | |
| Sodium dihydrogen phosphate | 0.9 | |
| Mannitol | 24.7 | |
| PVP K30 | 2.5 | 5.0 |
| Aqueous sodium hydroxide solution (1 N) | Proper amount | |
| Aqueous hydrochloric acid solution (1 N) | Proper amount | |
| Water for injection | qs1.0 mL | |

### Preparation:

(1) The formula amounts of tween 20, disodium hydrogen phosphate, sodium dihydrogen phosphate, mannitol and PVP K30 were measured out, added to water for injection that was about 60% of the total amount of the preparation, and dissolved and dispersed by stirring.
(2) The formula amount of the crystal form A of cariprazine mono-1-hydroxy-2-naphthoate dihydrate was added, and optionally, the pH was adjusted to 4.0-9.0 with sodium hydroxide or hydrochloric acid. The mixtures were brought to volume and completely dispersed by stirring to obtain suspensions of Examples 32 and 33.

The syringeability, suspendibility, settling ratio and redispersibility of the formula samples prepared in Examples 32 and 33 were investigated, and the investigation revealed that the suspension samples described above could all pass through 0.45 × 15 mm syringe needles and had good suspendibility, and the samples of Examples 32 and 33 had settling ratios greater than 0.9 for at least 10 min and good redispersibility (the suspension samples could be rapidly dispersed by shaking after settling).

### Examples 34-37: Aqueous Suspensions of Cariprazine Mono-1-hydroxy-2-naphthoate of Different Particle Sizes

| Component | Amount (mg) |
|---|---|
| | Example 34/Example 35/Example 36/Example 37 |
| Crystal form C of cariprazine mono-1-hydroxy-2-naphthoate (Example 4) | 650 |
| Tween 20 | 12.5 |
| Disodium hydrogen phosphate | 22.5 |
| Sodium dihydrogen phosphate | 4.5 |
| Mannitol | 123.5 |
| Sodium carboxymethylcellulose | 12.5 |
| Water for injection | qs5.0 mL |

### Preparation:

(1) The formula amounts of tween 20, disodium hydrogen phosphate, sodium dihydrogen phosphate and mannitol were measured out, added to water for injection that was about 60% of the total amount of the preparation, and dissolved and dispersed by stirring.
(2) The formula amount of the crystal form C of cariprazine mono-1-hydroxy-2-naphthoate was added to obtain aqueous suspensions of coarse particles.
(3) The aqueous suspensions of coarse particles obtained above in Examples 34-37 were ground and dispersed with a ball mill.
(4) The formula amount of sodium carboxymethylcellulose was added to the suspensions described above and completely dispersed by stirring. The mixtures were brought to volume to obtain suspensions of Examples 34-37 having a pH of 7.4±0.2.
(5) The particle size distribution of the samples of Examples 34-37 was measured on an OMEC LS-909 particle size analyzer, and the results are shown in the table below.

| Example | Dv10 (µm) | Dv50 (µm) | Dv90 (µm) |
|---|---|---|---|
| Example 34 | 6.107 | 16.977 | 36.631 |
| Example 35 | 4.502 | 9.977 | 18.639 |
| Example 36 | 0.755 | 2.193 | 9.973 |
| Example 37 | 0.683 | 1.531 | 7.104 |

From the results in the table above, it can be seen that in aqueous suspensions of the same formula, aqueous suspensions of particles of different particle sizes (Dv90) can be prepared by controlling the grinding parameters.

### Examples 38-40: Aqueous Suspensions of Different Crystal Forms of Cariprazine Mono-1-hydroxy-2-naphthoate

| Name | Amount (mg) | | |
|---|---|---|---|
| | Example 38 | Example 39 | Example 40 |
| Crystal form A of cariprazine mono-1-hydroxy-2-naphthoate dihydrate (Example 2) | 137 | - | - |
| Crystal form B of cariprazine mono-1-hydroxy-2-naphthoate hydrate (Example 3) | - | 130 | - |
| Crystal form C of cariprazine mono-1-hydroxy-2-naphthoate (Example 4) | - | - | 130 |
| Tween 20 | 5 | | |
| Disodium hydrogen phosphate | 4.5 | | |
| Sodium dihydrogen phosphate | 0.9 | | |
| Mannitol | | 24.7 | |
| Sodium carboxymethylcellulose | | 7.5 | |
| Water for injection | | Qs1.0 mL | |

### Preparation:

(1) The formula amounts of tween 20, disodium hydrogen phosphate, sodium dihydrogen phosphate, mannitol and sodium carboxymethylcellulose were measured out, added to water for injection that was about 60% of the total amount of the preparation, and dissolved and dispersed by stirring.
(2) The formula amounts of the crystal forms described above of cariprazine mono-1-hydroxy-2-naphthoate were added, and optionally, the pH was adjusted to 4.0-9.0 with sodium hydroxide or hydrochloric acid. The mixtures were brought to volume and completely dispersed by stirring to obtain suspensions of Examples 38-40.

The syringeability, suspendibility, settling ratio and redispersibility of the formula samples prepared in Examples 38-40 were investigated, and the investigation revealed that the suspension samples described above could all pass through 0.45 × 15 mm syringe needles and had good suspendibility, and the samples of Examples 38-40 had settling ratios greater than 0.9 for at least 10 min and good redispersibility (the suspension samples could be rapidly dispersed by shaking after settling).

According to the results in the table above and the investigation of syringeability, suspendibility, settling ratio and wettability, aqueous suspensions of the same formula apply to different crystal forms of cariprazine mono-1-hydroxy-2-naphthoate.

### Example 41: Investigation of Stability of Aqueous Suspension of Cariprazine 1-hydroxy-2-naphthoate at 60 °C

The 0-day, 5-day and 10-day related substances of the aqueous suspension of the crystal form A of cariprazine mono-1-hydroxy-2-naphthoate dihydrate prepared in Example 21 and the aqueous suspension of the crystal form A of cariprazine bis-1-hydroxy-2-naphthoate hydrate prepared in Example 24 were detected at 60 °C, and the results are shown in the table below.

| Example | Time point | Total impurity (%) | Example | Time point | Total impurity (%) |
|---|---|---|---|---|---|
| 21 | Day 0 | 0.30 | 24 | Day 0 | 0.57 |
| | Day 5 | 0.32 | | Day 5 | 0.78 |
| | Day 10 | 0.34 | | Day 10 | 1.09 |

From the results in the table above, it can be seen that in aqueous suspensions of the same formula, the aqueous suspension of the crystal form A of cariprazine mono-1-hydroxy-2-naphthoate dihydrate was more stable than the aqueous suspension of the crystal form A of cariprazine bis-1-hydroxy-2-naphthoate hydrate.

### Examples 42-43: Amplification and Stability of Aqueous Suspensions of Cariprazine Mono-1-hydroxy-2-naphthoate of Different Formulas

| Component | Amount (g) | |
|---|---|---|
| | Example 42 | Example 43 |
| Crystal form A of cariprazine mono-1-hydroxy-2-naphthoate dihydrate (Example 2) | 27.44 | |
| Tween 20 | 1 | |
| Disodium hydrogen phosphate | 0.9 | |
| Sodium dihydrogen phosphate | 0.18 | |
| Mannitol | 6.5 | |
| Sodium carboxymethylcellulose | 1.5 | / |
| PVP K30 | / | 1 |
| Water for injection | Qs200.0 mL | |

### Preparation:

(1) The formula amounts of tween 20, disodium hydrogen phosphate, sodium dihydrogen phosphate, mannitol and sodium carboxymethylcellulose/PVPK30 were measured out, added to water for injection that was about 60% of the total amount of the preparation, and dissolved and dispersed by stirring, and the mixtures were brought to 200 mL;
(2) the formula amount of the crystal form A of cariprazine mono-1-hydroxy-2-naphthoate dihydrate was added to obtain aqueous suspensions of coarse particles, and the aqueous suspensions were brought to 200 mL;
(3) the aqueous suspensions of coarse particles obtained above in Examples 42 and 43 were ground and dispersed with a sand mill; and
(4) the particle size distribution of the samples of Examples 42 and 43 was measured on an OMEC LS-909 particle size analyzer, and the results are shown in the table below.

| Example | Dv10 (µm) | Dv50 (µm) | Dv90 (µm) | Dv100 (µm) |
|---|---|---|---|---|
| Example 42 | 0.686 | 0.936 | 1.618 | 3.664 |
| Example 43 | 0.675 | 0.986 | 2.166 | 4.552 |

From the results in the table above, it can be seen that aqueous suspensions of particles having a smaller particle size (Dv90) can be prepared by controlling the grinding parameters.

The suspensions prepared in the examples described above were placed for the stability under the conditions of high temperature (60 °C), illumination, acceleration (40 °C/75% RH), long term (25 °C/60% RH), low temperature (2-8 °C) and the like. The data on related substances, content, particle size and the like are shown in the table below.

| Example | Condition | Content (%) | Maximum single impurity (%) | Total impurity (%) | Particle size (mean, µm) | | | |
|---|---|---|---|---|---|---|---|---|
| | | | | | Dv10 | Dv50 | Dv90 | Dv100 |
| 42 | 0 d | 97.3 | 0.79 | 1.09 | 0.686 | 0.936 | 1.618 | 3.664 |
| | 60 °C-5 d | 98.0 | 0.75 | 1.01 | 0.622 | 0.984 | 1.885 | 4.239 |
| | 60 °C-10 d | 100.8 | 0.71 | 1.04 | 0.568 | 0.882 | 1.654 | 3.375 |
| | 60 °C-1 M | 101.4 | 0.65 | 1.14 | 0.700 | 1.001 | 1.772 | 3.377 |
| | Low temperature-1 M | 102.7 | 0.79 | 1.13 | 0.722 | 0.977 | 1.785 | 3.657 |
| | Long term-1 M | 103.4 | 0.76 | 1.10 | 0.697 | 1.057 | 1.767 | 2.675 |
| | Acceleration-1 M | 101.5 | 0.70 | 1.04 | 0.721 | 1.035 | 1.573 | 3.375 |
| | Acceleration-2 M | 99.0 | 0.64 | 0.97 | 0.684 | 0.940 | 1.770 | 4.226 |
| | Illumination-5 d | 101.1 | 0.64 | 1.09 | 0.713 | 1.005 | 1.394 | 2.635 |
| | Illumination-10 d | 98.7 | 0.61 | 1.26 | 0.660 | 0.901 | 1.468 | 2.673 |
| 43 | 0 d | 105.1 | 0.80 | 1.11 | 0.675 | 0.986 | 2.166 | 4.552 |
| | 60 °C-5 d | 105.4 | 0.77 | 1.03 | 0.693 | 0.987 | 1.828 | 3.380 |
| | 60 °C-10 d | 101.8 | 0.72 | 1.06 | 0.590 | 0.888 | 1.769 | 3.363 |
| | 60 °C-1 M | 106.5 | 0.67 | 1.14 | 0.683 | 1.003 | 2.001 | 3.924 |
| | Low temperature-1 M | 103.0 | 0.79 | 1.13 | 0.821 | 1.279 | 1.967 | 3.365 |
| | Long term-1 M | 103.0 | 0.77 | 1.11 | 0.690 | 0.957 | 1.732 | 2.675 |
| | Acceleration-1 M | 105.1 | 0.71 | 1.05 | 0.685 | 0.956 | 1.795 | 3.069 |
| | Acceleration-2 M | 104.8 | 0.67 | 1.00 | 0.687 | 0.959 | 1.882 | 3.368 |
| | Illumination-5 d | 101.9 | 0.65 | 1.10 | 0.572 | 0.92 | 1.959 | 5.333 |
| | Illumination-10 d | 104.5 | 0.62 | 1.27 | 0.700 | 0.964 | 1.752 | 2.675 |

The data described above show that the sanding samples of different formulas had good stability in the aspects of related substances, content, particle size and the like under different conditions, and meanwhile, the stability samples described above had good syringeability and redispersibility and were not abnormal.

### Example 44: Hydrochloride Dissociation Study

The crystal form I of cariprazine hydrochloride prepared in Example 8 was taken and separately added to corresponding aqueous media of pH 6, pH 7, pH 7.4, pH 8, pH 9, etc. The mixtures were shaken at 37 °C for 4 h and centrifuged. The residues were tested by XRPD, and the results show that the crystal form I of cariprazine hydrochloride dissociated into cariprazine free base, and the pH 7.4 comparison results are shown in FIG. 16 (others not shown). As can be seen from the results, the crystal form I of cariprazine hydrochloride in the solution is unstable, the efficacy is changed due to the change in the crystal form after administration, and the medication safety is reduced.

### Examples 45-47: Pharmacokinetic Study of Formula for Cariprazine 1-hydroxy-2-naphthoate Suspension in Rats

| Component | Amount (mg) | | | |
|---|---|---|---|---|
| | Example 42 | Example 45 | Example 46 | Example 47 |
| Crystal form A of cariprazine mono-1-hydroxy-2-naphthoate dihydrate (Example 2) | 27.44 g | 685 | - | - |
| Crystal form A of cariprazine bis-1-hydroxy-2-naphthoate hydrate (Example 5) | - | - | 850 | - |
| Amorphous cariprazine embonate (Example 7) | - | - | - | 573 |
| Poloxamer 188 | - | 5 | | - |
| Tween 20 | 1 g | - | | 100 |
| Disodium hydrogen phosphate | 0.9 g | 22.5 | | 45 |
| Sodium dihydrogen phosphate | 0.18 g | 4.5 | | 9 |
| Mannitol | 6.5 g | 123.5 | | 247 |
| Sodium carboxymethylcellulose | 1.5 g | 37.5 | | 50 |
| Water for injection | Qs200.0 mL | qs5.0 mL | | qs10.0 mL |

For the process of preparing the injectable suspension of Example 42, reference was made to Example 42 described above.
1. Process of preparing injectable suspensions of Examples 45-46:
   (1) The formula amounts of poloxamer 188, disodium hydrogen phosphate, sodium dihydrogen phosphate, mannitol and sodium carboxymethylcellulose were measured out, added to water for injection that was about 60% of the total amount of the preparation, and dissolved and dispersed by stirring.
   (2) The formula amounts of corresponding samples of the crystal form A of cariprazine mono-1-hydroxy-2-naphthoate dihydrate or the crystal form A of cariprazine bis-1-hydroxy-2-naphthoate hydrate were added and completely dispersed by stirring. The mixtures were brought to volume to obtain injectable suspensions of Examples 45-46 having a pH of 7.4±0.2.
2. Process of preparing injectable suspension of Example 47:
   (1) The formula amounts of tween 20, disodium hydrogen phosphate, sodium dihydrogen phosphate, mannitol and sodium carboxymethylcellulose were measured out, added to water for injection that was about 60% of the total amount of the preparation, and dissolved and dispersed by stirring.
   (2) The formula amount of amorphous cariprazine embonate was added and completely dispersed by stirring. The mixture was brought to volume to obtain the injectable suspension of Example 47 having a pH of 7.4±0.2.
3. The particle size distribution of the samples of Examples 45-46 was measured on an OMEC LS-909 particle size analyzer, and the results are shown in the table below.

| Example | Dv10 (µm) | Dv50 (µm) | Dv90 (µm) |
|---|---|---|---|
| Example 45 | 7.625 | 15.837 | 38.015 |
| Example 46 | 5.847 | 14.654 | 32.237 |

### Example 48: Preparation of Oral Liquid of Crystal Form I of Hydrochloride

(1) A proper amount of the sample of the crystal form I of cariprazine hydrochloride obtained in Example 8 was added to water for injection that was about 60% of the total amount of the preparation, and optionally, 0.1% tween 20 and 0.35% sodium carboxymethylcellulose were added. The mixture was stirred and dispersed.
(2) The pH was adjusted to 5.0 to 5.5 with hydrochloric acid, and the mixture was brought to volume to obtain the final clear oral liquid of Example 48.

### Example 49: Pharmacokinetic Experiment

Examples 45-48: 12 male SD rats were divided into four groups, of which three groups were given single doses of formula samples of cariprazine mono-1-hydroxy-2-naphthoate (Example 45), cariprazine bis-1-hydroxy-2-naphthoate (Example 46) and cariprazine embonate (Example 47) by intramuscular injection at 9 mg/kg, and plasma was collected at 0 h, 1 h, 3 h, 7 h, 24 h, 4 d, 7 d, 15 d, 20 d, 25 d and 30 d after administration; rats in group 4 were orally and intragastrically given single doses of the formula sample of crystal form I of cariprazine hydrochloride (Example 48) at 0.3 mg/kg, and plasma was collected 5 min, 15 min, 30 min, 1 h, 2 h, 3 h, 4 h, 6 h, 8 h, 12 h and 24 h after administration. In the whole study, the animals in the intramuscular injection group were given ad libitum access to food and water, and those in the oral intragastrical group were fasted overnight before administration and given access to food at 4 h after administration.

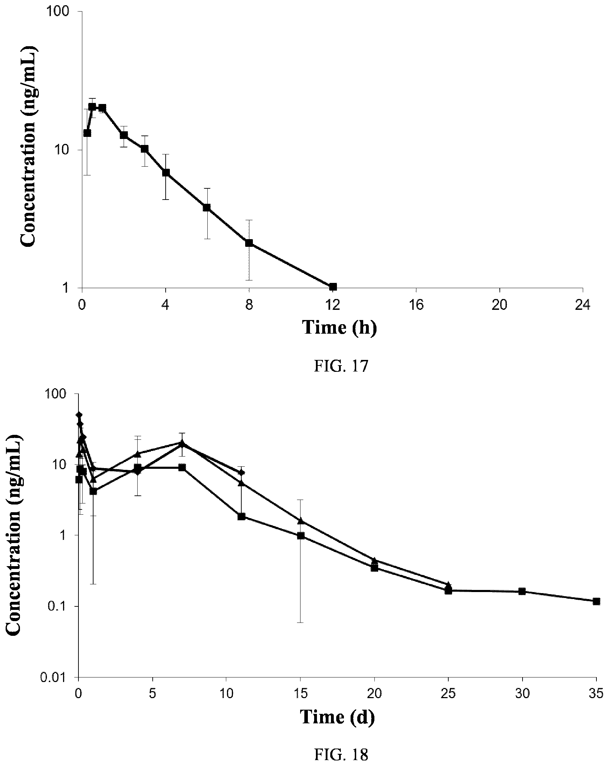

Collection of plasma sample: about 150 µL of blood was collected from the jugular vein (whole blood was centrifuged within 30 min to separate plasma) and placed in a tube containing anticoagulant EDTA-K2, and after treatment, plasma was stored in a freezer at -70 °C before use. After pretreatment of the plasma sample, 1 µL of the solution was analyzed by LC-MS/MS. The pharmacokinetic parameters in animals are shown in Tables 4-7, and the drug concentration-time curves are shown in FIGs. 17-19.

The results show that the absorbed plasma concentration of the cariprazine hydrochloride oral formula group reached the peak value Cmax of 20 ng/mL within 1 h after administration and decreased to 1 ng/mL at 12 h, and the mean residence time (MRTlast) was only 2.75 h; compared to the oral group, the three injection groups of Examples 45, 46 and 47 showed significant increases in time to peak (Tmax) and mean residence time (MRTlast), Tmax increased from hours to days, and MRTlast values were 4.5 days, 6.5 days and 5.4 days, respectively, which were 39, 57 and 47 times that of the oral experimental group, indicating significant sustained-release effects.

Example 42: 3 male SD rats and 3 female rats each constituted one group, for a total of two groups. Rats in the two groups were given single doses of the formula sample of cariprazine mono-1-hydroxy-2-naphthoate (Example 42) by intramuscular injection at 9 mg/kg, and plasma was collected at 1 h, 3 h, 7 h, 24 h, 4 d, 7 d, 10 d, 13 d, 16 d, 19 d, 22 d, 25 d and 28 d after administration. In the whole study, the animals were given ad libitum access to food and water. Collection of plasma sample: after being collected, the blood sample was placed on ice and centrifuged (centrifugation conditions: 6800 g, 6 min, 2-8 °C) within 1 h to separate plasma. The plasma was placed in a tube containing anticoagulant EDTA-K2, and the plasma sample was stored in a freezer at -80 °C before analysis. After pretreatment of the plasma sample, the solution was analyzed by LC-MS/MS.

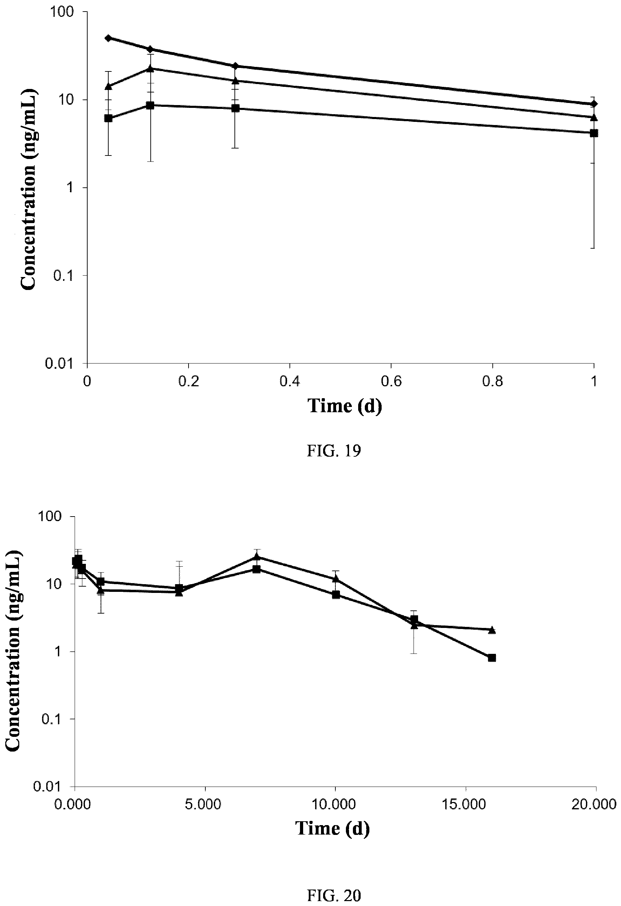

The pharmacokinetic parameters in animals are shown in Tables 8-9, and the drug concentration-time curves are shown in FIGs. 20-21.

The results show that compared to the oral group (Example 48, the absorbed plasma concentration reached the peak value Cmax of 20 ng/mL within 1 h and decreased to 1 ng/mL at 12 h, and the mean residence time (MRTlast) was only 2.75 h), the two injection groups of rats of different sexes for Example 42 showed significant increases in time to peak (Tmax) and mean residence time (MRTlast), Tmax increased from hours to days, and MRTlast values were 5.9 days and 6.5 days, respectively, which were 51 and 57 times that of the oral experimental group, indicating significant sustained-release effects.

The Cmax values (26 ng/mL (male), 29 ng/mL (female), 13 ng/mL and 24 ng/mL) of the injection groups of Examples 42, 45 and 46 were not significantly different from that in the oral group, indicating that the preparations prepared in this patent does not cause the safety hazards such as adverse effects of the medicaments and the like caused by excessive plasma concentrations after administration; the Cmax value of the injection group of Example 47 was relatively high, which was 50.5 ng/mL, indicating that there may be some safety hazards such as adverse effects.

In addition, as can be seen from the maintenance of the plasma concentration, the injection groups of Examples 42, 45 and 46 maintained at about 1 ng/mL within 15-20 days after administration, while the group of Example 47 could only maintain for 11 days, indicating that the samples of Examples 42, 45 and 46 have longer time for exerting the efficacy *in vivo* and better sustained-release effects compared to Example 47.

Further, the injection groups of Examples 42, 45 and 46 had AUClast values of 138 day*ng/mL, 68 day*ng/mL and 168 day*ng/mL, respectively, in male rats, indicating that the samples of Examples 42 and 46 have an advantage in bioavailability over Example 45.

**Table 4. Pharmacokinetic parameters of suspension of crystal form A of cariprazine mono-1-hydroxy-2-naphthoate dihydrate (Example 45) after intramuscular injection in rats**

| Pharmacokineti c parameter | Unit | Rat #1 | Rat #2 | Rat #3 | Mean | Standard deviation SD | Coefficient of variation (%) |
|---|---|---|---|---|---|---|---|
| Tₘₐₓ | day | 4.00 | 0.292 | 0.125 | 1.47 | 2.19 | 149 |
| Cₘₐₓ | ng/mL | 24.3 | 3.21 | 11.5 | 13.0 | 10.6 | 81.7 |
| T_{1/2} | day | 4.09 | NA | 2.50 | 3.30 | NA | NA |
| AUCₗₐₛₜ | day*ng/mL | 177 | 3.48 | 23.9 | 68.3 | 95.1 | 139 |
| AUC_{INF} | day*ng/mL | 178 | NA | 25.2 | 102 | NA | NA |
| MRT_{INF} | day | 5.93 | NA | 3.78 | 4.85 | NA | NA |
| MRTₗₐₛₜ | day | 5.79 | NA | 3.18 | 4.49 | NA | NA |

**Table 5. Pharmacokinetic parameters of suspension of crystal form A of cariprazine bis-1-hydroxy-2-naphthoate dihydrate (Example 46) after intramuscular injection in rats**

| Pharmacokinet ic parameter | Unit | Rat #1 | Rat #2 | Rat #3 | Mean | Standard deviation SD | Coefficient of variation (%) |
|---|---|---|---|---|---|---|---|
| Tₘₐₓ | day | 0.125 | 0.125 | 7.00 | 2.42 | 3.97 | 164 |
| Cₘₐₓ | ng/mL | 15.4 | 34.5 | 22.1 | 24.0 | 9.69 | 40.4 |
| T_{1/2} | day | 2.04 | 1.32 | 2.47 | 1.94 | 0.583 | 30.0 |
| AUCₗₐₛₜ | day*ng/mL | 106 | 223 | 174 | 168 | 58.8 | 35.0 |
| AUC_{INF} | day*ng/mL | 107 | 224 | 175 | 169 | 59.0 | 35.0 |
| MRT_{INF} | day | 6.44 | 5.17 | 7.99 | 6.53 | 1.42 | 21.7 |
| MRTₗₐₛₜ | day | 6.37 | 5.13 | 7.91 | 6.47 | 1.39 | 21.5 |

**Table 6. Pharmacokinetic parameters of suspension of amorphous cariprazine embonate (Example 47) after intramuscular injection in rats**

| Pharmacokinetic parameter | Unit | Rat # 1 | Rat #2 | Rat #3 | Mean | Standard deviation SD | Coefficient of variation (%) |
|---|---|---|---|---|---|---|---|
| Tₘₐₓ | day | 0.042 | 0.042 | 0.042 | 0.042 | 0.00 | 0.00 |
| Cₘₐₓ | ng/mL | 61.5 | 47.5 | 42.4 | 50.5 | 9.89 | 19.6 |
| T_{1/2} | day | 3.41 | 12.0 | 29.9 | 15.1 | 13.5 | 89.6 |
| AUCₗₐₛₜ | day*ng/m L | 181 | 111 | 128 | 140 | 36.6 | 26.2 |
| AUC_{INF} | day*ng/m L | 191 | 290 | 572 | 351 | 198 | 56.3 |
| MRT_{INF} | day | 5.37 | 19.6 | 43.3 | 22.8 | 19.2 | 84.3 |
| MRTₗₐₛₜ | day | 4.77 | 5.53 | 5.96 | 5.42 | 0.603 | 11.1 |

**Table 7. Pharmacokinetic parameters of oral liquid of crystal form I of cariprazine hydrochloride (Example 48) after oral administration to rats**

| Pharmacokin etic parameter | Unit | Rat #1 | Rat #2 | Rat #3 | Mea n | Standard deviation SD | Coefficient of variation (%) |
|---|---|---|---|---|---|---|---|
| Tₘₐₓ | hr | 1.00 | 0.50 0 | 0.50 0 | 0.66 7 | 0.289 | 43.3 |
| Cₘₐₓ | ng/mL | 18.7 | 23.7 | 19.8 | 20.7 | 2.63 | 12.7 |
| T_{1/2} | hr | 1.97 | 2.60 | 2.49 | 2.36 | 0.336 | 14.3 |
| AUCₗₐₛₜ | hr*ng/ mL | 50.7 | 85.6 | 75.7 | 70.7 | 18.0 | 25.4 |
| AUC_{INF} | hr*ng/ mL | 53.6 | 89.3 | 86.0 | 76.3 | 19.8 | 25.9 |
| MRT_{INF} | hr | 2.75 | 3.69 | 3.84 | 3.43 | 0.594 | 17.3 |
| MRTₗₐₛₜ | hr | 2.29 | 3.16 | 2.79 | 2.75 | 0.433 | 15.8 |

**Table 8. Pharmacokinetic parameters of suspension of crystal form A of cariprazine mono-1-hydroxy-2-naphthoate dihydrate (Example 42) after intramuscular injection in male rats**

| Pharmacokin etic parameter | Unit | Rat # 1 | Rat #2 | Rat #3 | Mean | Standard deviation SD | Coefficient of variation (%) |
|---|---|---|---|---|---|---|---|
| Tₘₐₓ | hr | 3.0 | 1.0 | 7.0 | 3.67 | 3.06 | 83.32 |
| Cₘₐₓ | ng/mL | 18.07 | 39.81 | 20.85 | 26.24 | 11.83 | 45.07 |
| T_{1/2} | day | 2.22 | 2.33 | 1.86 | 2.14 | 0.24 | 11.42 |
| AUCₗₐₛₜ | day*ng/ mL | 115.43 | 142.77 | 155.31 | 137.8 4 | 20.40 | 14.80 |
| AUC_{obs} | day*ng/ mL | 118.59 | 150.51 | 156.97 | 142.0 3 | 20.55 | 14.47 |
| MRTₗₐₛₜ | day | 6.55 | 4.82 | 6.19 | 5.85 | 0.91 | 15.55 |
| MRT_{obs} | day | 6.88 | 5.42 | 6.32 | 6.21 | 0.74 | 11.93 |

**Table 9. Pharmacokinetic parameters of suspension of crystal form A of cariprazine mono-1-hydroxy-2-naphthoate dihydrate (Example 42) after intramuscular injection in female rats**

| Pharmacokin etic parameter | Unit | Rat #1 | Rat #2 | Rat #3 | Mean | Standard deviation SD | Coefficient of variation (%) |
|---|---|---|---|---|---|---|---|
| Tₘₐₓ | hr | 168.0 | 1.0 | 168.0 | 112.3 3 | 96.42 | 85.83 |
| Cₘₐₓ | ng/mL | 24.46 | 33.40 | 29.02 | 28.96 | 4.47 | 15.44 |
| T_{1/2} | day | 2.02 | 1.17 | 2.11 | 1.77 | 0.52 | 29.53 |
| AUCₗₐₛₜ | day*ng/ mL | 150.39 | 132.07 | 226.83 | 169.7 6 | 50.26 | 29.61 |
| AUC_{obs} | day*ng/ mL | 153.48 | 133.14 | 236.45 | 174.3 5 | 54.73 | 31.39 |
| MRTₗₐₛₜ | day | 6.68 | 5.53 | 7.22 | 6.48 | 0.86 | 13.33 |
| MRT_{obs} | day | 6.92 | 5.61 | 7.71 | 6.75 | 1.06 | 15.73 |

The embodiments of the technical solutions of the present disclosure have been described above by way of example. It should be understood that the protection scope of the present disclosure is not limited to the embodiments described above. Any modification, equivalent replacement, improvement, and the like made by those skilled in the art without departing from the spirit and principle of the present disclosure shall fall within the protection scope of the claims of the present application.

## Claims

1. A pharmaceutically acceptable salt of cariprazine, wherein the pharmaceutically acceptable salt is selected from cariprazine mono-1 -hydroxy-2-naphthoate represented by formula (I) shown below, cariprazine bis-1-hydroxy-2-naphthoate represented by formula (II) shown below, or cariprazine embonate represented by formula (III) shown below: 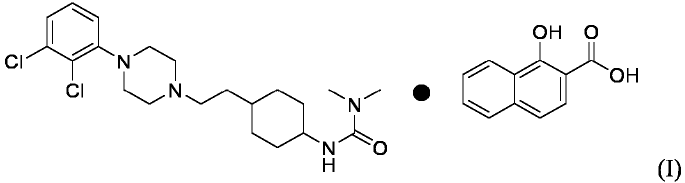 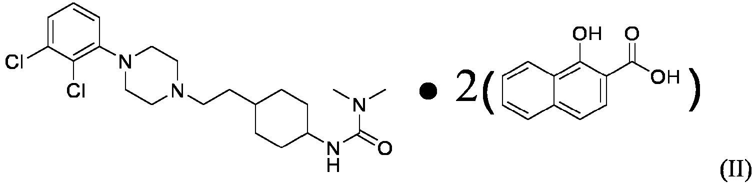 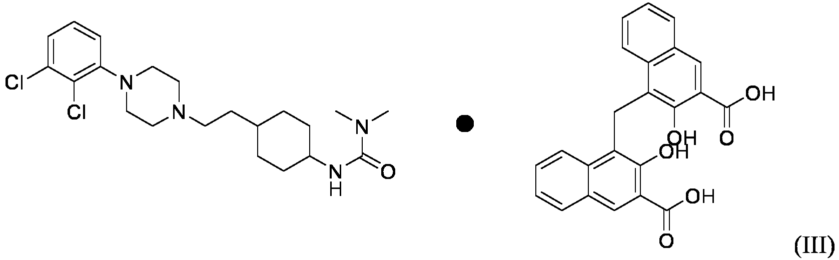 preferably, the pharmaceutically acceptable salt is selected from at least one of the following forms:
crystal form A of cariprazine mono-1-hydroxy-2-naphthoate, wherein the crystal form A of cariprazine mono-1-hydroxy-2-naphthoate, preferably crystal form A of cariprazine mono-1-hydroxy-2-naphthoate hydrate, and more preferably crystal form A of cariprazine mono-1-hydroxy-2-naphthoate dihydrate, has an X-ray powder diffraction pattern comprising characteristic peaks at 20 values of 15.6°±0.2°, 19.7°±0.2°, 20.7°±0.2°, etc.;
preferably, the crystal form A of cariprazine mono-1-hydroxy-2-naphthoate is the crystal form A of cariprazine mono-1-hydroxy-2-naphthoate hydrate, more preferably the crystal form A of cariprazine mono-1-hydroxy-2-naphthoate dihydrate;
a single crystal of the crystal form A of cariprazine mono-1-hydroxy-2-naphthoate dihydrate, wherein the single crystal has an X-ray diffraction pattern comprising characteristic peaks at 20 values of 17.5°±0.2°, 19.7°±0.2°, 20.8°±0.2°, etc.;
crystal form B of cariprazine mono-1-hydroxy-2-naphthoate, wherein the crystal form B of cariprazine mono-1-hydroxy-2-naphthoate has an X-ray powder diffraction pattern comprising characteristic peaks at 20 values of 14.6°±0.2°, 18.8°±0.2°, 19.5°±0.2°, etc.;
crystal form C of cariprazine mono-1-hydroxy-2-naphthoate, wherein the crystal form C of cariprazine mono-1-hydroxy-2-naphthoate has an X-ray powder diffraction pattern comprising characteristic peaks at 20 values of 17.8°±0.2°, 19.9°±0.2°, 21.1°±0.2°, etc.;
an amorphous form of cariprazine bis-1-hydroxy-2-naphthoate;
a crystal form of cariprazine bis-1-hydroxy-2-naphthoate, such as crystal form A of cariprazine bis-1-hydroxy-2-naphthoate, wherein the crystal form A of cariprazine bis-1-hydroxy-2-naphthoate has an X-ray powder diffraction pattern comprising characteristic peaks at 20 values of 4.7°±0.2°, 9.4°±0.2°, 20.8°±0.2°, etc.;
preferably, the crystal form A of cariprazine bis-1-hydroxy-2-naphthoate is crystal form A of cariprazine bis-1-hydroxy-2-naphthoate hydrate;
an amorphous form of cariprazine embonate; and
crystal form H of cariprazine embonate, wherein the crystal form H of cariprazine embonate has an X-ray powder diffraction pattern comprising characteristic peaks at 20 values of 7.3°±0.2°, 8.8°±0.2°, 9.4°±0.2°, 14.6°±0.2°, 17.4°±0.2°, 18.1°±0.2°, 18.6°±0.2°, 19.2°±0.2°, 22.9°±0.2°, etc.

2. The pharmaceutically acceptable salt according to claim 1, wherein the crystal form A of cariprazine mono-1-hydroxy-2-naphthoate, preferably the crystal form A of cariprazine mono-1-hydroxy-2-naphthoate hydrate, and more preferably the crystal form A of cariprazine mono-1-hydroxy-2-naphthoate dihydrate, has an X-ray powder diffraction pattern comprising characteristic peaks at 20 values of 12.7°±0.2°, 15.6°±0.2°, 17.3°±0.2°, 19.7°±0.2°, 20.2°±0.2°, 20.7°±0.2°, etc.;
preferably, the crystal form A of cariprazine mono-1-hydroxy-2-naphthoate, preferably the crystal form A of cariprazine mono-1-hydroxy-2-naphthoate hydrate, and more preferably the crystal form A of cariprazine mono-1-hydroxy-2-naphthoate dihydrate, has an X-ray powder diffraction pattern comprising characteristic peaks at 20 values of 12.7°±0.2°, 15.6°±0.2°, 17.3°±0.2°, 18.8°±0.2°, 19.7°±0.2°, 20.2°±0.2°, 20.7°±0.2°, 24.3°±0.2°, etc.;
more preferably, the crystal form A of cariprazine mono-1-hydroxy-2-naphthoate, preferably the crystal form A of cariprazine mono-1-hydroxy-2-naphthoate hydrate, and more preferably the crystal form A of cariprazine mono-1-hydroxy-2-naphthoate dihydrate, has an X-ray powder diffraction pattern comprising absorption peaks at 20 values of 4.4°±0.2°, 8.6°±0.2°, 9.7°±0.2°, 11.4°±0.2°, 12.7°±0.2°, 15.6°±0.2°, 16.5°±0.2°, 16.9°±0.2°, 17.3°±0.2°, 18.8°±0.2°, 19.7°±0.2°, 20.2°±0.2°, 20.7°±0.2°, 23.3°±0.2°, 24.3°±0.2°, 24.9°±0.2°, 29.5°±0.2°, etc.;
more preferably, the crystal form A of cariprazine mono-1-hydroxy-2-naphthoate, preferably the crystal form A of cariprazine mono-1-hydroxy-2-naphthoate hydrate, and more preferably the crystal form A of cariprazine mono-1-hydroxy-2-naphthoate dihydrate, has an X-ray powder diffraction pattern substantially as shown in FIG. 1;
preferably, the crystal form A of cariprazine mono-1-hydroxy-2-naphthoate, preferably the crystal form A of cariprazine mono-1-hydroxy-2-naphthoate hydrate, and more preferably the crystal form A of cariprazine mono-1-hydroxy-2-naphthoate dihydrate, has a differential scanning calorimetry pattern substantially as shown in FIG. 2;
preferably, the crystal form A of cariprazine mono-1-hydroxy-2-naphthoate, preferably the crystal form A of cariprazine mono-1-hydroxy-2-naphthoate hydrate, and more preferably the crystal form A of cariprazine mono-1-hydroxy-2-naphthoate dihydrate, has a melting point of about 101.3 °C;
preferably, the crystal form A of cariprazine mono-1-hydroxy-2-naphthoate, preferably the crystal form A of cariprazine mono-1-hydroxy-2-naphthoate hydrate, and more preferably the crystal form A of cariprazine mono-1-hydroxy-2-naphthoate dihydrate, has a thermogravimetric analysis pattern substantially as shown in FIG. 2;
preferably, the single crystal has an X-ray diffraction pattern comprising characteristic peaks at 20 values of 17.5°±0.2°, 19.0°±0.2°, 19.7°±0.2°, 20.3°±0.2°, 20.8°±0.2°, 23.4°±0.2°, etc.;
preferably, the single crystal has an X-ray diffraction pattern comprising characteristic peaks at 20 values of 12.7°±0.2°, 15. 7°±0.2°, 17.5°±0.2°, 19.0°±0.2°, 19.7°±0.2°, 20.3°±0.2°, 20.8°±0.2°, 23.4°±0.2°, etc.;
more preferably, the single crystal has an X-ray diffraction pattern comprising absorption peaks at 20 values of 11.5°±0.2°, 12.7°±0.2°, 15.7°±0.2°,, 16.6°±0.2°, 16.9°±0.2°, 17.5°±0.2°, 18.1°±0.2°, 19.0°±0.2°, 19.7°±0.2°, 20.0°±0.2°, 20.3°±0.2°, 20.8°±0.2°, 21.3°±0.2°, 23.4°±0.2°, 23.6°±0.2°, 24.4°±0.2°, 24.9°±0.2°, 29.6°±0.2°, etc.

3. The pharmaceutically acceptable salt according to claim 1 or 2, wherein the crystal form B of cariprazine mono-1-hydroxy-2-naphthoate has an X-ray powder diffraction pattern comprising characteristic peaks at 20 values of 14.6°±0.2°, 18.8°±0.2°, 19.5°±0.2°, etc.;
preferably, the crystal form B of cariprazine mono-1-hydroxy-2-naphthoate has an X-ray powder diffraction pattern comprising characteristic peaks at 20 values of 5.5°±0.2°, 11.5°±0.2°, 14.6°±0.2°, 18.8°±0.2°, 19.5°±0.2°, 23.7°±0.2°, etc.;
more preferably, the crystal form B of cariprazine mono-1-hydroxy-2-naphthoate has an X-ray powder diffraction pattern comprising characteristic peaks at 20 values of 5.5°±0.2°, 11.5°±0.2°, 13.0°±0.2°, 14.6°±0.2°, 16.9°±0.2°, 18.8°±0.2°, 19.5°±0.2°, 23.7°±0.2°, etc.;
more preferably, the crystal form B of cariprazine mono-1-hydroxy-2-naphthoate has an X-ray powder diffraction pattern comprising characteristic peaks at 20 values of 5.5°±0.2°, 9.2°±0.2°, 11.1°±0.2°, 11.5°±0.2°, 13.0°±0.2°, 13.9°±0.2°, 14.6°±0.2°, 16.6°±0.2°, 16.9°±0.2°, 18.5°±0.2°, 18.8°±0.2°, 19.5°±0.2°, 19.9°±0.2°, 20.3°±0.2°, 21.2°±0.2°, 23.7°±0.2°, 24.3°±0.2°, etc.;
more preferably, the crystal form B of cariprazine mono-1-hydroxy-2-naphthoate has an X-ray powder diffraction pattern substantially as shown in FIG. 4;
preferably, the crystal form B of cariprazine mono-1-hydroxy-2-naphthoate has a differential scanning calorimetry pattern substantially as shown in FIG. 5;
preferably, the crystal form B of cariprazine mono-1-hydroxy-2-naphthoate has a melting point of about 106.8 °C.

4. The pharmaceutically acceptable salt according to any one of claims 1-3, wherein the crystal form C of cariprazine mono-1-hydroxy-2-naphthoate has an X-ray powder diffraction pattern comprising characteristic peaks at 2θ values of 4.4°±0.2°, 15.6°±0.2°, 17.8°±0.2°, 19.9°±0.2°, 21.1°±0.2°, 23.7°±0.2°, etc.;
preferably, the crystal form C of cariprazine mono-1-hydroxy-2-naphthoate has an X-ray powder diffraction pattern comprising characteristic peaks at 20 values of 4.4°±0.2°, 12.8°±0.2°, 15.6°±0.2°, 17.8°±0.2°, 19.9°±0.2°, 20.2°±0.2°, 21.1°±0.2°, 23.7°±0.2°, etc.;
more preferably, the crystal form C of cariprazine mono-1-hydroxy-2-naphthoate has an X-ray powder diffraction pattern comprising characteristic peaks at 20 values of 4.4°±0.2°, 8.6°±0.2°, 9.7°±0.2°, 11.5°±0.2°, 12.8°±0.2°, 13.2°±0.2°, 15.6°±0.2°, 16.6°±0.2°, 17.8°±0.2°, 19.3°±0.2°, 19.9°±0.2°, 20.2°±0.2°, 21.1°±0.2°, 22.4°±0.2°, 23.7°±0.2°, 24.2°±0.2°, 24.9°±0.2°, etc.;
more preferably, the crystal form C of cariprazine mono-1-hydroxy-2-naphthoate has an X-ray powder diffraction pattern substantially as shown in FIG. 7;
preferably, the crystal form C of cariprazine mono-1-hydroxy-2-naphthoate has a differential scanning calorimetry pattern substantially as shown in FIG. 8;
preferably, the crystal form C of cariprazine mono-1-hydroxy-2-naphthoate has a melting point of about 103.4 °C.

5. The pharmaceutically acceptable salt according to any one of claims 1-4, wherein the crystal form A of cariprazine bis-1-hydroxy-2-naphthoate has an X-ray powder diffraction pattern comprising characteristic peaks at 20 values of 4.7°±0.2°, 9.4°±0.2°, 12.7°±0.2°, 20.1°±0.2°, 20.8°±0.2°, 27.4°±0.2°, etc.;
preferably, the crystal form A of cariprazine bis-1-hydroxy-2-naphthoate has an X-ray powder diffraction pattern comprising characteristic peaks at 20 values of 4.7°±0.2°, 9.4°±0.2°, 12.7°±0.2°, 17.1°±0.2°, 19.1°±0.2°, 20.1°±0.2°, 20.8°±0.2°, 27.4°±0.2°, etc.;
more preferably, the crystal form A of cariprazine bis-1-hydroxy-2-naphthoate has an X-ray powder diffraction pattern comprising characteristic peaks at 20 values of 4.7°±0.2°, 8.8°±0.2°, 9.4°±0.2°, 12.7°±0.2°, 16.5°±0.2°, 17.1°±0.2°, 18.4°±0.2°, 19.1°±0.2°, 19.9°±0.2°, 20.1°±0.2°, 20.8°±0.2°, 21.1°±0.2°, 21.9°±0.2°, 24.1°±0.2°, 24.8°±0.2°, 25.6°±0.2°, 27.4°±0.2°, etc.;
more preferably, the crystal form A of cariprazine bis-1-hydroxy-2-naphthoate has an X-ray powder diffraction pattern substantially as shown in FIG. 10;
preferably, the crystal form A of cariprazine bis-1-hydroxy-2-naphthoate has a differential scanning calorimetry pattern substantially as shown in FIG. 11;
preferably, the crystal form A of cariprazine bis-1-hydroxy-2-naphthoate has a melting point of about 86.3 °C;
preferably, the crystal form A of cariprazine bis-1-hydroxy-2-naphthoate has a thermogravimetric analysis pattern substantially as shown in FIG. 11.

6. The pharmaceutically acceptable salt according to any one of claims 1-5, wherein the crystal form H of cariprazine embonate has an X-ray powder diffraction pattern substantially as shown in FIG. 13;
alternatively, cariprazine embonate is an amorphous form, which preferably has an X-ray powder diffraction pattern substantially as shown in FIG. 14.

7. A preparation method for the pharmaceutically acceptable salt according to any one of claims 1-5, comprising one of preparation methods selected from the following:
(1) a preparation method for the crystal form A of cariprazine mono-1-hydroxy-2-naphthoate, preferably the crystal form A of cariprazine mono-1-hydroxy-2-naphthoate hydrate, and more preferably the crystal form A of cariprazine mono-1-hydroxy-2-naphthoate dihydrate, comprising the following steps:
(a1) dissolving cariprazine in an aqueous phosphoric acid solution and filtering to obtain solution A; mixing an aqueous 1-hydroxy-2-naphthoic acid solution with an aqueous sodium hydroxide solution and filtering to obtain solution B; and
(a2) adding the solution B to the solution A according to a solute molar ratio of 1:1 and stirring at room temperature to obtain the crystal form A of cariprazine mono-1-hydroxy-2-naphthoate, preferably the crystal form A of cariprazine mono-1-hydroxy-2-naphthoate hydrate, and more preferably the crystal form A of cariprazine mono-1-hydroxy-2-naphthoate dihydrate;
(2) a preparation method for the crystal form A of cariprazine mono-1-hydroxy-2-naphthoate, preferably the crystal form A of cariprazine mono-1-hydroxy-2-naphthoate hydrate, and more preferably the crystal form A of cariprazine mono-1-hydroxy-2-naphthoate dihydrate, comprising the following steps:
(a3) mixing cariprazine and 1-hydroxy-2-naphthoic acid with a solvent according to a molar ratio of 1:1; and
(a4) stirring at 25-70 °C to obtain the crystal form A of cariprazine mono-1-hydroxy-2-naphthoate, preferably the crystal form A of cariprazine mono-1-hydroxy-2-naphthoate hydrate, and more preferably the crystal form A of cariprazine mono-1-hydroxy-2-naphthoate dihydrate; wherein preferably, the solvent is a mixed system of methanol, ethanol or acetone and water;
preferably, methanol, ethanol or acetone and water can be in a volume ratio of 2:1 to 1:2, such as 1:1;
preferably, cariprazine and the total volume of the solvent system are in a mass-to-volume ratio of 1 g:(5-30) mL, such as 1 g:(5-20) mL, e.g., 1 g:10 mL;
(3) a preparation method for the crystal form B of cariprazine mono-1-hydroxy-2-naphthoate, comprising the following steps:
(b1) mixing cariprazine and 1-hydroxy-2-naphthoic acid with a solvent according to a molar ratio of 1:1; and
(b2) stirring at 25-70 °C to obtain the crystal form B of cariprazine mono-1-hydroxy-2-naphthoate;
wherein preferably, the solvent is a mixed system of methanol, ethanol or acetone and water;
preferably, methanol, ethanol or acetone and water can be in a volume ratio of 2:1 to 1:2, such as 1.5:1.
preferably, cariprazine and the total volume of the solvent system are in a mass-to-volume ratio of 1 g:(0.5-10) mL, such as 1 g:(0.5-1.5) mL, e.g., 1 g:1 mL;
(4) a preparation method for the crystal form C of cariprazine mono-1-hydroxy-2-naphthoate, comprising the following steps:
(c1) mixing cariprazine and 1-hydroxy-2-naphthoic acid with a solvent according to a molar ratio of 1:1; and
(c2) stirring at 25-70 °C to obtain the crystal form C of cariprazine mono-1-hydroxy-2-naphthoate;
wherein preferably, the solvent is a mixed system of methanol, ethanol or acetone and water;
preferably, methanol, ethanol or acetone and water can be in a volume ratio of 2:1 to 1:2, such as 1.5:1.
preferably, cariprazine and the total volume of the solvent system are in a mass-to-volume ratio of 1 g:(5-30) mL, such as 1 g:(5-20) mL, e.g., 1 g:10 mL;
(5) a preparation method for the crystal form A of cariprazine bis-1-hydroxy-2-naphthoate, comprising the following steps:
adding cariprazine and 1-hydroxy-2-naphthoic acid to a solvent according to a molar ratio of 1:2 and stirring at 25-70 °C to obtain the crystal form A of cariprazine bis-1-hydroxy-2-naphthoate; wherein preferably, the solvent is a mixed system of methanol, ethanol or acetone and water;
preferably, methanol, ethanol or acetone and water can be in a volume ratio of 2:1 to 1:2, such as 1:1;
preferably, cariprazine and the total volume of the solvent system are in a mass-to-volume ratio of 1 g:(5-40) mL, such as 1 g:(10-30) mL, e.g., 1 g:20 mL.

8. A combination of pharmaceutically acceptable salts of cariprazine, wherein the combination comprises:
at least one, e.g., two or three, of the pharmaceutically acceptable salts according to any one of claims 1-6; and
other pharmaceutically acceptable salts of cariprazine, or other forms of cariprazine mono-1-hydroxy-2-naphthoate, cariprazine bis-1-hydroxy-2-naphthoate or cariprazine embonate, which are optionally present or absent;
preferably, based on the total weight of the pharmaceutically acceptable salts of cariprazine in the combination, the content of the crystal forms of cariprazine mono-1-hydroxy-2-naphthoate or cariprazine bis-1-hydroxy-2-naphthoate according to any one of claims 1-5 in percentages by weight is greater than the content of the other salt forms or crystal forms of cariprazine;
for example, based on the total weight of the pharmaceutically acceptable salts of cariprazine in the combination, the content of the crystal forms of cariprazine mono-1-hydroxy-2-naphthoate or cariprazine bis-1-hydroxy-2-naphthoate according to any one of claims 1-5 in percentages by weight is greater than 80%, preferably greater than 90%, and more preferably greater than 95% or 99%.

9. A pharmaceutical composition comprising the pharmaceutically acceptable salt according to any one of claims 1-6, preferably comprising one of cariprazine mono-1-hydroxy-2-naphthoate, cariprazine bis-1-hydroxy-2-naphthoate, cariprazine embonate or the crystal forms thereof, wherein
preferably, the pharmaceutical composition comprises solid particles of cariprazine that have particle sizes Dv(10) of ≤ 30 microns, Dv(50) of ≤ 50 microns and Dv(90) of ≤ 100 microns, preferably ≤ 50 microns; wherein the solid particles of cariprazine can be selected from one of cariprazine mono-1-hydroxy-2-naphthoate, cariprazine bis-1-hydroxy-2-naphthoate, cariprazine embonate or the crystal forms thereof according to any one of claims 1-6;
preferably, the pharmaceutical composition of cariprazine can comprise the following components:
(a) cariprazine 1-hydroxy-2-naphthoate;
(b) sodium carboxymethylcellulose or PVP K30;
(c) tween 20 or poloxamer 188;
(d) disodium hydrogen phosphate;
(e) sodium dihydrogen phosphate; and
(f) mannitol;
and, optionally, the pharmaceutical composition of cariprazine can comprise a pH regulator, such as sodium hydroxide or hydrochloric acid;
preferably, the pharmaceutical composition of cariprazine can have a pH of 4.0-9.0;
more preferably, the pharmaceutical composition of cariprazine is a suspension or a suspension agent, preferably an aqueous suspension or an aqueous suspension agent;
more preferably, the pharmaceutical composition of cariprazine is an injection, such as a long-acting injection;
for example, in the pharmaceutical composition or injection of cariprazine, the solid particles of cariprazine are at a concentration of no less than 30 mg/mL, such as 30-120 mg/mL, preferably 90 mg/mL (w/v).

10. Use of one of the pharmaceutically acceptable salt according to any one of claims 1-6, the combination of the pharmaceutically acceptable salts of cariprazine according to claim 8 or the pharmaceutical composition according to claim 9 in the manufacture of a medicament, wherein the medicament is used for treating and/or preventing a cognitive disorder or a mental disorder, such as psychosis, bipolar disorder or acute mania.
